(19) Europäisches Patentamt
European Patent Office
Office européen des brevets

(11) EP 1 321 486 A1

(12) EUROPÄISCHE PATENTANMELDUNG

(43) Veröffentlichungstag:
**25.06.2003 Patentblatt 2003/26**

(51) Int Cl.[7]: **C08G 73/02**, A61L 15/26

(21) Anmeldenummer: **02025781.2**

(22) Anmeldetag: **16.11.2002**

(84) Benannte Vertragsstaaten:
**AT BE BG CH CY CZ DE DK EE ES FI FR GB GR IE IT LI LU MC NL PT SE SK TR**
Benannte Erstreckungsstaaten:
**AL LT LV MK RO SI**

(30) Priorität: **07.12.2001 DE 10160097**

(71) Anmelder: **Sandler AG**
**95126 Schwarzenbach/Saale (DE)**

(72) Erfinder:
- **Die Erfinder haben auf ihre Nennung verzichtet**

(54) **Modifizierte Poly-2-oxazoline, ihre Herstellung und ihre Verwendung als Sorbentien und Flüssigkeitsabsorber**

(57) Die Erfindung betrifft neuartige modifizierte Poly-2-oxazoline, ihre Herstellung und ihre Vernetzung sowie ihre Verwendung als oder zur Herstellung von Sorbentien und Flüssigkeitsabsorbern. Die erfindungsgemäßen Homopolymeren und Copolymeren weisen ein Poly-ethylenimin-Grundgerüst auf, das partiell oder vollständig N-substituiert ist, wobei die N-Substituenten bevorzugt ethylenisch ungesättigt und damit vernetzbar sind.

Die erfindungsgemäßen Polymeren können durch kationische ring-öffnende Polymerisation entsprechender 2-substituierter 2-Oxazoline oder durch polymeranaloge Umsetzung von Polyethyleniminen, die gegebenenfalls modifiziert sind, hergestellt werden.

Die vernetzten Polymeren stellen hydrophile Superabsorber dar, die vorteilhaft in flüssigkeitsabsorbierenden Einwegartikeln, wie Windeln, Slipeinlagen oder Inkontinenzhilfsmitteln, verwendbar sind.

EP 1 321 486 A1

## Beschreibung

Die Erfindung betrifft neue substituierte und funktionalisierte hydrophile Homopolymere und Copolymere, besonders auf der Basis von 2-substituierten 2-Oxazolinen (2-substituierten $\Delta_2$-1-ox-3-azolinen), die entsprechend ein von Polyethylenimin abgeleitetes Grundgerüst aufweisen, Verfahren zu ihrer Herstellung sowie ihre Verwendung als und in Sorbentien und Absorbern, insbesondere in flüssigkeitsabsorbierenden Einwegartikeln, wie sie vor allem im Hygienebereich eingesetzt werden.

Die Erfindung betrifft dabei insbesondere mit vernetzbaren, ungesättigten Gruppen funktionalisierte Polymere, die sich durch Vernetzung zu einem quellbaren Netzwerk umsetzen lassen, das gute Absorbereigenschaften aufweist.

Zunächst wird der Teil des Erfindungskonzepts erläutert, der sich auf die Polymeren und ihre Herstellung bezieht. Dem polymerbezogenen Gegenstand der Erfindung liegt die Aufgabe zugrunde, neue Homopolymere und Copolymere auf der Basis eines von Polyethylenimin abgeleiteten Grundgerüsts anzugeben, die hydrophil sind und vorzugsweise radikalisch vernetzbare funktionelle Gruppen aufweisen und durch Vernetzung Hydrogele mit gutem Quellvermögen in Wasser und wässerigen Systemen ergeben. Ferner sollen Verfahren zur Herstellung dieser Polymeren und ihre Verwendung als oder in Sorbentien und Absorbern angegeben werden.

Die Aufgabe wird gemäß den entsprechenden unabhängigen Ansprüchen gelöst. Die abhängigen Ansprüche betreffen vorteilhafte Ausführungsformen des Konzepts der Erfindung.

Die Erfindung betrifft gemäß dem allgemeinsten Aspekt Homopolymere und Copolymere, die aus folgenden Einheiten A und/oder B bestehen oder diese Einheiten enthalten:

(A) 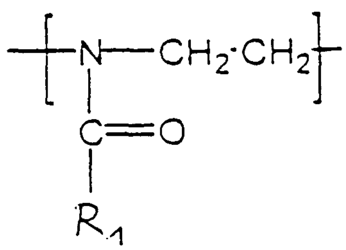

und/oder

(B) 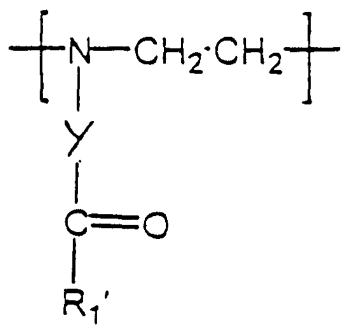

,

wobei bedeuten:

- $R_1$ Alkyl, insbesondere niederes Alkyl, oder eine hydrophile Gruppe,
- $R_1'$ eine radikalisch vernetzbare ethylenisch ungesättigte Gruppe oder eine Gruppe, die eine radikalisch vernetzbare ethylenisch ungesättigte Gruppe aufweist,

und

Y eine Einfachbindung

oder
eine Gruppe der Formel $-CO-(CH_2)_p-O-$ oder $-(CH_2)_p-O-$ mit p = 2 bis 7, die jeweils mit dem C-Ende am N-Atom gebunden ist.

In den Einheiten A bedeutet die Gruppe $R_1$ vorzugsweise $C_{1-6}$-Alkyl, insbesondere Methyl, Ethyl, Propyl oder Butyl.

Hydrophile Gruppen $R_1$ sind vorzugsweise Hydroxyalkyl, insbesondere $C_{1-6}$-Hydroxyalkyl und vor allem im Hydroxymethyl, Hydroxyethyl und Hydroxypropyl $HO-CH_2CH_2-CH_2$-, Polyoxyethylengruppen $[O-CH_2-CH_2]_b$ OH mit b im Bereich von 1 bis 20, die an der CO-Gruppe gebunden sind,
und
Alkylgruppen, die eine unsubstituierte oder substituierte Ammoniumgruppe tragen, insbesondere Ammoniomethyl $R_3\overset{\oplus}{N}-CH_2$-, Ammonioethyl $R_3\overset{\oplus}{N}-CH_2-CH_2$- und Ammoniopropyl $R_3\overset{\oplus}{N}-(CH_2)_3$- mit R = H oder Alkyl, besonders niederes $C_{1-6}$-Alkyl.

Weitere hydrophile Gruppen sind etwa von Zuckern abgeleitete Reste.

In den Einheiten B bedeutet die Gruppe $R_1$ vorzugsweise Vinyl $H_2C=CH$-, 1-Methylvinyl $H_2C=C(CH_3)$-, 1-Ethylvinyl $H_2C=C(C_2H_5)$-, Propenyl, Allyl oder eine von Zimtsäure

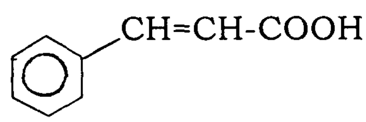

abgeleitete Gruppe, die über eine Esterbindung an der Carboxylgruppe, an einem der C-Atome der ungesättigten Bindung oder am Phenylring gebunden ist.

Grundsätzlich kommen für die Gruppen $R_1$' alle Gruppen in Frage, die eine radikalisch vernetzbare ethylenisch ungesättigte Gruppe aufweisen.

Um eine Vernetzung zu ermöglichen, sollen die erfindungsgemäßen Polymeren mindestens zwei vernetzbare ungesättigte Gruppen im Molekül aufweisen.

Unabhängig vom Vorliegen von ungesättigten Gruppen $R_1$' können auch ungesättigte vernetzbare Gruppen in der Polymerhauptkette sowie in Form von Endgruppen vorliegen, wie später erläutert wird.

Erfindungsgemäße Copolymere des oben definierten allgemeinen Typs weisen vorteilhaft die Formel I auf,

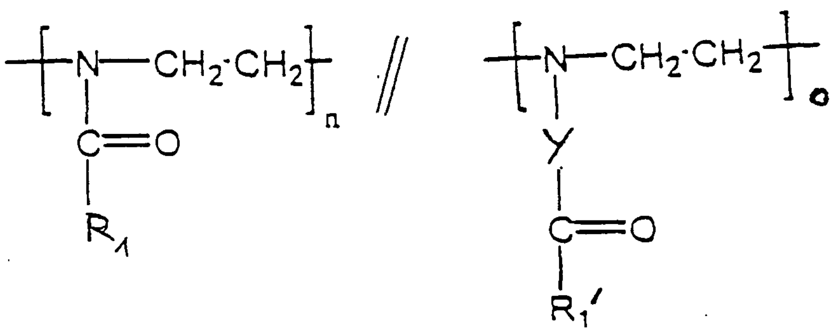

(I),

(A) (B)

worin bedeuten:

| | |
|---|---|
| $R_1$, $R_1$' und Y | dasselbe wie oben definiert, |
| n | 5 bis 300, vorzugsweise 15 bis 100, und |
| o | 2 bis 50, vorzugsweise 3 bis 20, |

wobei der Doppelstrich // bedeutet, dass die Einheiten A und B in den Polymerketten statistisch verteilt oder in Form von Blöcken beliebiger Abfolge vorliegen.

Die Polymeren des oben definierten Typs können Endgruppen beliebiger Art aufweisen.

Je nach der bei der Herstellung angewandten Verfahrensweise können statistische Copolymere oder Blockcopolymere erhalten werden.

Die Polymerisationsgrade n und o geben im Fall von statistischen Copolymeren den mittleren Anteil an entsprechenden Einheiten A und B im Polymer und im Fall von Blockcopolymeren die entsprechende mittlere Blocklänge bzw. bei mehreren Blöcken A und/oder B im Polymermolekül die Summe der mittleren Blocklängen an. Dies gilt analog auch für die anderen erfindungsgemäßen Copolymeren.

Die erfindungsgemäßen Copolymeren können neben den Einheiten A und/oder B Einheiten C der folgenden Formel in der Hauptkette aufweisen:

$$(C) \qquad \{NH\text{-}CH_2\text{-}CH_2\},$$

also Ethylenimin-Einheiten.

Erfindungsgemäße ternäre Blockcopolymere aus den Einheiten A, B und C haben die Formel II, wobei Endgruppen außeracht gelassen sind:

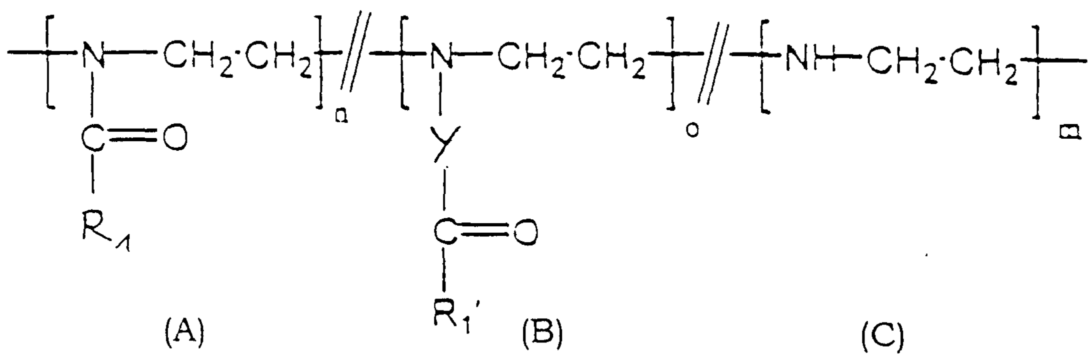

(II),

worin bedeuten:

| | |
|---|---|
| $R^1$, $R_1$' und Y | dasselbe wie oben angegeben, |
| m | 2 bis 25, vorzugsweise 3 bis 10, |
| n | 5 bis 300, vorzugsweise 15 bis 100, und |
| o | 2 bis 50, vorzugsweise 3 bis 20 und insbesondere 3 bis 10. |

Die Doppelstriche // bedeuten auch hierbei, dass die Einheiten A, B und C in den Polymerketten statistisch verteilt oder in Form von Blöcken beliebiger Abfolge vorliegen.

In den Formeln der erfindungsgemäßen Polymeren bedeuten $R_1$ bevorzugt Methyl oder Ethyl und $R_1$' Vinyl, 1-Methylvinyl oder 1-Ethylvinyl.

Die Endgruppen der erfindungsgemäßen Homopolymeren und Copolymeren unterliegen keiner Beschreibung. Vorteilhafte Endgruppen sind H, OH, $NH_2$, mono- und disubstituiertes $NH_2$, besonders mit $C_{1-6}$-Alkyl-Substituenten, Acyl, Acyloxy, Alkoxy mit $C_{1-6}$-Alkyl oder Phenoxy oder substituiertes Phenoxy.

Solche Endgruppen und ihre Anbringung am Polymermolekül sind dem Fachmann per se geläufig.

Besonders bevorzugt sind Endgruppen, die mindestens eine ungesättigte Gruppe aufweisen, vorteilhaft eine ethylenisch ungesättigte Gruppe.

Nicht einschränkende Beispiele für solche günstigen ungesättigten Endgruppen sind

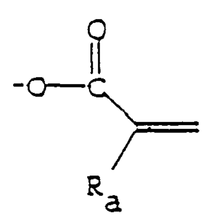

,

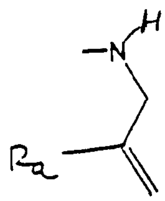

und

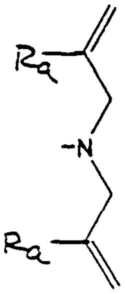

,

worin $R_a$ H, Methyl oder Ethyl bedeutet.

Diese Endgruppen haben den Vorteil, dass sie vernetzbar sind.

Im Rahmen der Erfindung können selbstverständlich auch Endgruppen vorliegen, die drei ungesättigte Gruppierungen aufweisen.

Wenn Y keine Einfachbindung bedeutet und $R_1$' Vinyl, 1-Methylvinyl oder 1-Ethylvinyl darstellt, können entsprechend in den erfindungsgemäßen Polymeren Einheiten der Formel

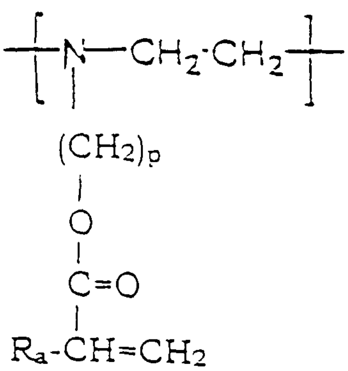

vorliegen, worin

$R_a$ H, Methyl oder Ethyl und
p eine ganze Zahl von 2 bis 7

bedeuten.

Ein vorteilhaftes Beispiel für vernetzbare Homopolymere mit ungesättigten Endgruppen sind Polymere der Formel IIIa,

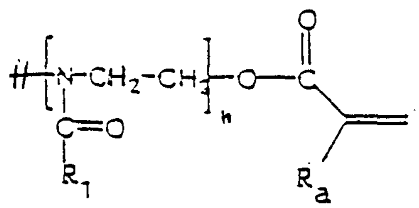 (IIIa)

und der Formel III

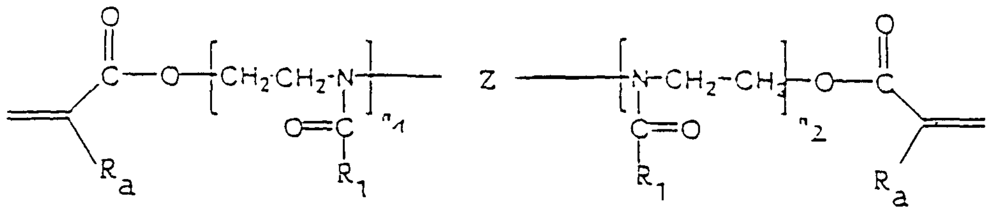 (III),

in der bedeuten:

$R_1$ dasselbe wie oben definiert,

$R_a$ H, Methyl oder Ethyl,

Z eine zweiwertige, von einem bifunktionellen kationischen Initiator abgeleitete Gruppe, insbesondere eine ungesättigte Gruppe und vorzugsweise die Gruppe $-CH_2-CH=CH-CH_2-$, und

$n_1$ und $n_2$ mittlere Polymerisationsgrade, deren Summe $n_1+n_2$ gleich n ist, wobei n 5 bis 300 bedeutet.

Die zweiwertige Gruppe Z ist im Rahmen der Erfindung allgemein eine Gruppe der Formel $-CH_2-R-CH_2-$, wobei R unter bifunktionellen Alkylgruppen, Arylgruppen, Alkengruppen, Alkingruppen, Heteroarylgruppen, Alkylethergruppen und Alkylestergruppen ausgewählt ist.

Gut geeignete Gruppen Z sind

$$-CH_2-CH=CH-CH_2- ,$$

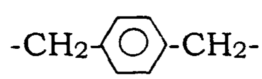 ,

$$-CH_2-(CH_2)_{1-4}-CH_2-$$

und

$$-CH_2-C \equiv C-CH_2- .$$

Unter bifunktionellen Initiatoren werden Verbindungen verstanden, die z.B. zur kationischen ringöffnenden Polymerisation von 2-substituierten 2-Oxazolinen wie etwa

(M) 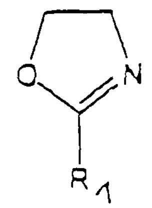

und

(N) 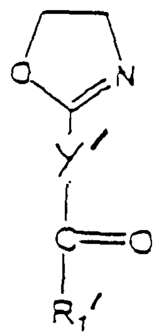

geeignet sind,
wobei $R_1$, $R_1$' und Y' wie oben definiert sind.

Solche Initiatoren sind beispielsweise Verbindungen der Formel

X-Z-X

mit X = Halogen, insbesondere Cl und Br,
ferner Triflate und Tosylate, wie z.B. Methyltriflat $H_3\overset{\oplus}{C}\overset{\ominus}{O}{}_3S\text{-}CF_3$ und Methyltosylat.

Ein gut geeigneter Initiator vom Typ X-Z-X ist trans-1,4-Dibrom-2-buten der Formel

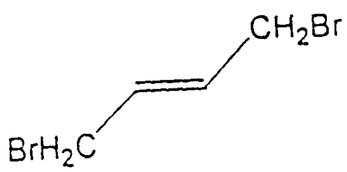

Durch Verwendung von solchen Initiatoren mit einer ungesättigten Gruppe wird in die Hauptkette des entstehenden Polymers mindestens eine ungesättigte und vernetzbare Gruppe eingeführt.

Ein weiterer geeigneter Initiator ist Methyltriflat.

Für den Fachmann ist klar, dass sich beliebige bifunktionelle Initiatoren und besonders Dihalogenverbindungen zur Initiierung der kationischen ringöffnenden Polymerisation von 2-substituierten 2-Oxazolinen eignen.

Homopolymere vom Typ der Formel III mit ungesättigten Endgruppen besitzen so beispielsweise die Formel IV

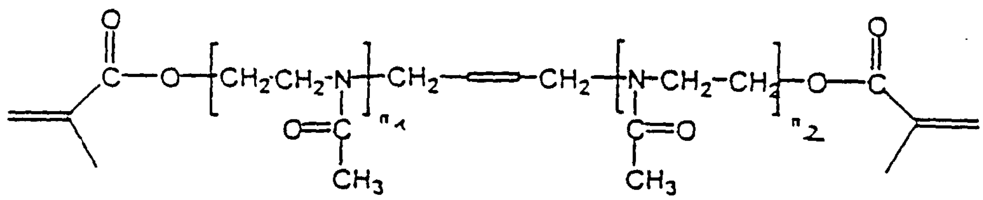

(IV),

worin $n_1$ und $n_2$ wie oben bei Formel III definiert sind.

Erfindungsgemäße Copolymere mit ungesättigten Endgruppen können die Formel V aufweisen,

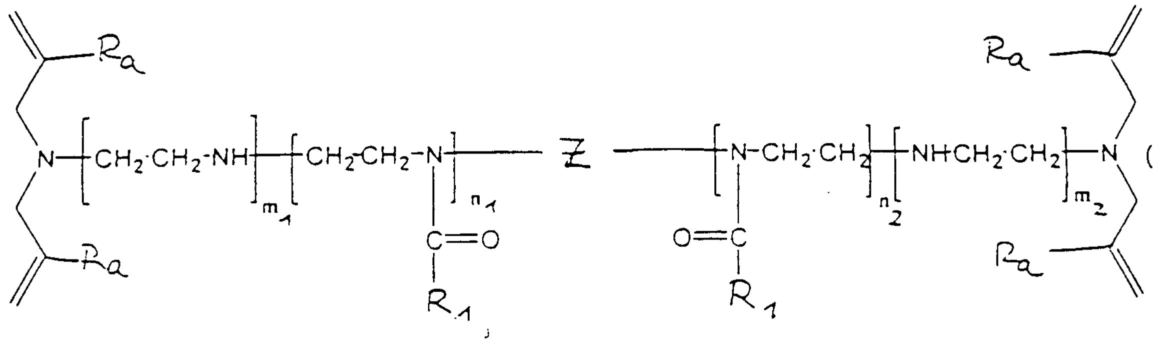

(V),

in der bedeuten:

| | |
|---|---|
| $R_1$, $R_a$, Z, $n_1$ und $n_2$ | dasselbe wie oben definiert und |
| $m_1$ und $m_2$ | mittlere Polymerisationsgrade, deren Summe $m_1$+$m_2$ gleich m ist, wobei m 2 bis 25 bedeutet. |

Bei den Copolymeren der Formel V liegen wie im Fall der Formel IV nur an den Kettenenden ungesättigte, vernetzbare Gruppen vor, wenn von der zentralen Gruppe Z abgesehen wird.

Die - gegebenenfalls gemäß $R_a$ substituierten - Diallylamino-Endgruppen, die durch eine Terminierungsreaktion in das entsprechende lebende kationische Polymer eingeführt werden können, haben den Vorteil, dass jeweils zwei vernetzbare ungesättigte Gruppen pro Endgruppe vorliegen, während die Acrylat- oder Alkylacrylat-Endgruppen des Homopolymers der Formel IV nur jeweils eine ungesättigte vernetzbare Gruppe besitzen.

Durch die Mengenanteile der beiden Comonomereinheiten lässt sich der Grad der Hydrophilie und damit das Quellungsvermögen in vernetztem Zustand in weiten Grenzen einstellen.

Ein vorteilhaftes Beispiel für erfindungsgemäße Copolymere vom Typ der Formel V sind Copolymere der Formel VI

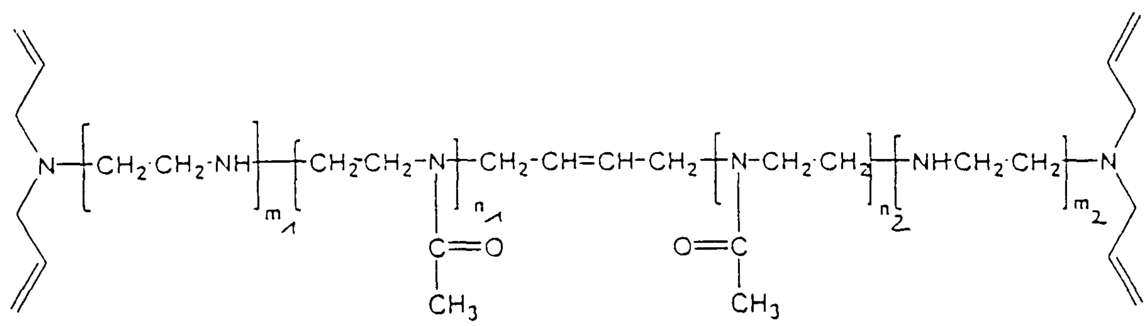

(VI)

mit $m_1$, $m_2$, $n_1$ und $n_2$ wie oben definiert,
die durch die Allylamino-Endgruppen und durch die vom 2-Buten abgeleitete, vom Initiator stammende Gruppe ungesättigt und vernetzbar sind. Bei diesen Verbindungen ist die Anzahl der vernetzbaren Gruppen entsprechend ebenso begrenzt wie in den Fällen der Polymeren der Formeln III, IV und V.

Zum Gegenstand der Erfindung gehören auch Copolymere der Formel VII,

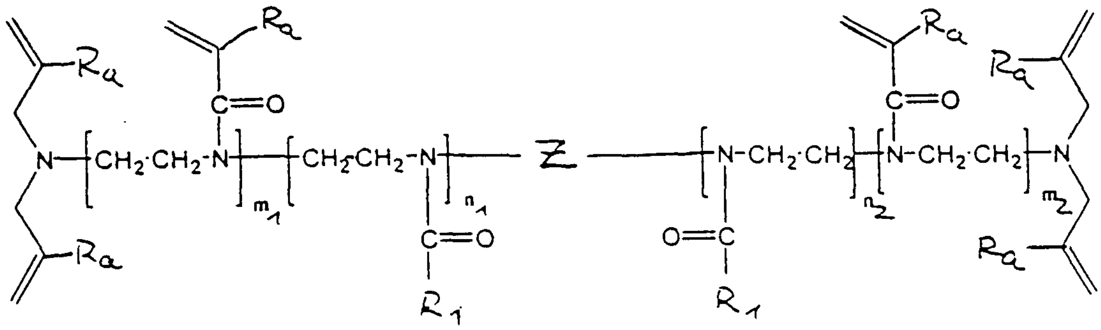

(VII),

in der $R_1$, $R_a$, Z, $m_1$, $m_2$, $n_1$ und $n_2$ die weiter oben angegebene Bedeutung besitzen.

Bei diesen Copolymeren liegen zusätzlich zu den vernetzbaren Endgruppen und gegebenenfalls auch einer ungesättigten Gruppe Z weitere ungesättigte und radikalisch vernetzbare Gruppen vor, die in diesem Fall Acryloyl- oder Alkylacryloyl-Gruppen sind. Diese Copolymeren können daher je nach den Werten von $m_1$ und $m_2$ eine mehr oder weniger große und bei der Herstellung einstellbare Anzahl ungesättigter vernetzbarer Gruppen aufweisen, was für die Einstellung der Quellfähigkeit entsprechender Copolymerer in wässerigen Systemen wichtig ist, besonders im Hinblick auf die Verwendung als Sorbentien und Flüssigkeitsabsorber.

Noch konkretere Beispiele für Copolymere des Typs der Formel VII sind Copolymere der Formel VIII,

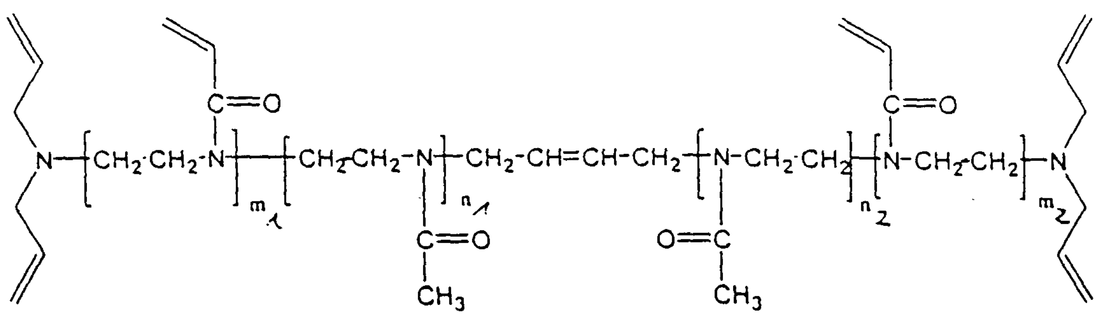

(VIII)

mit $m_1$, $m_2$, $n_1$ und $n_2$ wie oben definiert.

Die Gruppe Z liegt vor allem dann vor, wenn das entsprechende Polymer aus einem 2-substituierten 2-Oxazolin durch kationische ringöffnende Polymerisation erhalten ist, wie später im Detail erläutert wird.

Die erfindungsgemäßen Polymeren, welche die Gruppe Z enthalten, also etwa die Verbindungen der Formeln III bis VIII, sind nicht zwingend symmetrisch zu Z aufgebaut. Im Fall der Verbindungen der

Formel III bedeutet dies, dass $n_1$ und $n_2$ nicht notwendig gleich sein müssen.

Die Aufgabe von Polymerisationsgraden, wie $n_1$, $n_2$, etc., bedeutet ferner im Rahmen der Erfindung nicht notwendig, dass Blockcopolymere vorliegen, da auch eine statistische Verteilung der Einheiten vorliegen kann.

Typische Strukturelemente, welche als kettenendständige Gruppen die oben erläuterten Endgruppen aufweisen und/oder direkt oder durch eine oder mehrere Gruppen Z miteinander verbunden sein können, sind:

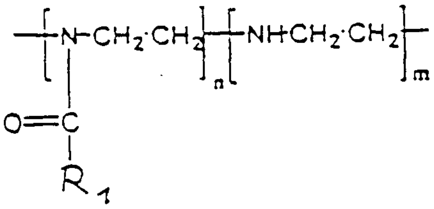

,

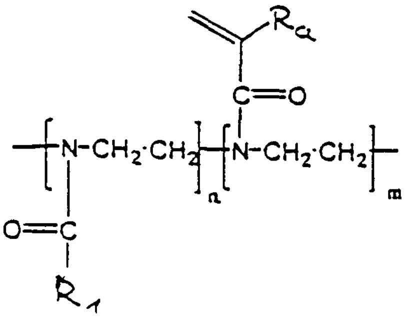

,

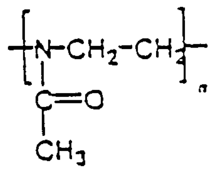

,

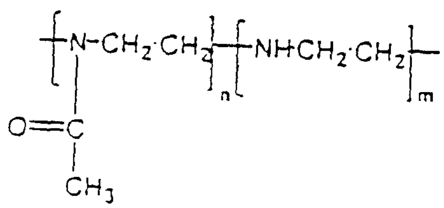

,

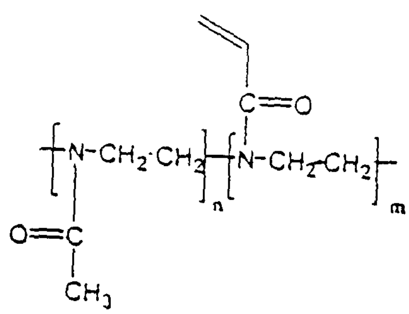

,

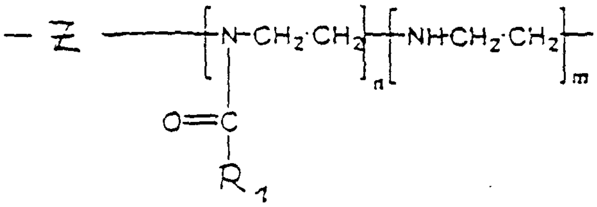

,

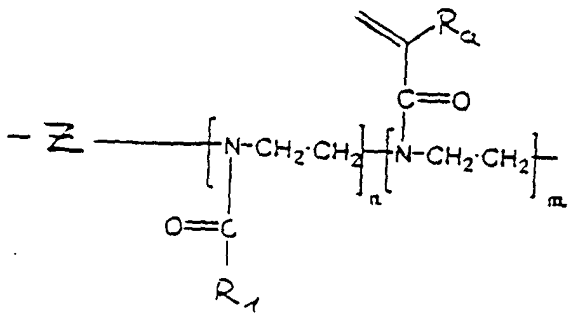

,

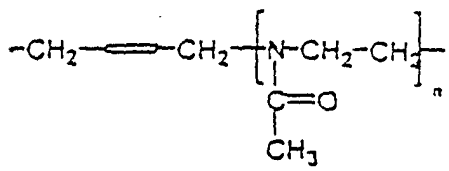

,

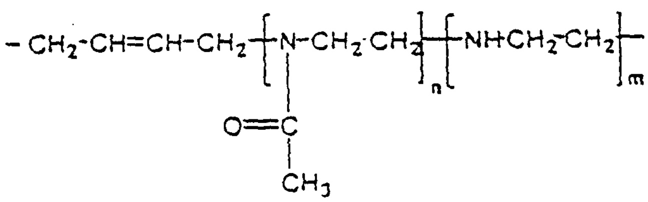

und

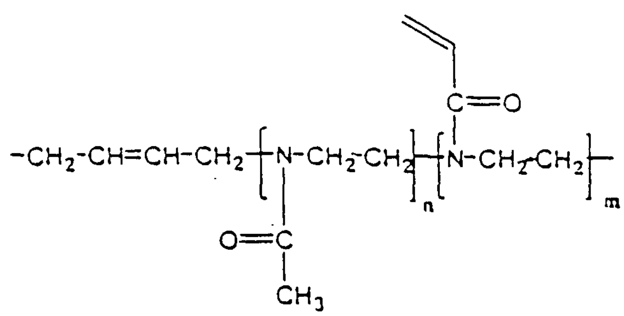

,

wobei $R_1$, $R_a$, Z, m und n wie oben definiert sind.

Die vorliegende Erfindung betrifft nach einem weiteren wesentlichen Aspekt teilweise oder vollständig vernetzte Homopolymere und Copolymere, die aus den oben erläuterten unvernetzten Homopolymeren und Copolymeren durch radikalisch initiierte Vernetzung erhalten sind.

Je nach dem Strukturtyp des entsprechenden Polymers liegen die Vernetzungsbrücken an einer Gruppe Z, an Endgruppen und/oder an Comonomereinheiten mit vernetzten Seitengruppen ($R_1$') vor.

Die teilvernetzten oder vollständig vernetzten Polymeren stellen hydrophile Produkte dar, die in Wasser und wässerigen Systemen Hydrogele bilden. Das Quellvermögen ist durch den Vernetzungsgrad einstellbar.

Als Ausgangsmaterialien für die Vernetzung besonders vorteilhaft sind unvernetzte erfindungsgemäße Polymere, die flüssig sind, da sie ohne Lösungsmittel oder mit nur geringen Lösungsmittelmengen auf Substrate, wie Vliese, aufgebracht und anschließend im aufgezogenen bzw. imprägnierten Zustand vernetzt werden können.

Von Vorteil ist hierbei, dass die erfindungsgemäßen Polymeren nicht nur auf hydrophile, sondern auch auf hydrophobe Substrate, besonders Vliese, aufgebracht und dann vernetzt werden können, da die Quellungseigenschaften auf hydrophoben Substraten, insbesondere Polyolefinsubstraten, besser sind als bei hydrophilen Trägern.

Diese Gegebenheit ist für die Herstellung von flüssigkeitsabsorbierenden Hygieneartikeln von besonderem Vorteil.

Zur Herstellung der erfindungsgemäßen Polymeren bestehen drei grundlegende Möglichkeiten:

(1) Die direkte kationische ringöffnende Polymerisation eines oder mehrerer 2-substituierter 2-Oxazoline mit anschließender Erzeugung geeigneter Endgruppen (Terminierung des lebenden Polymers);
(2) die polymeranaloge Umsetzung eines Polyethylenimins mit einem oder mehreren Modifizierungsreagentien, durch die gegebenenfalls funktionelle (ungesättigte) Gruppen eingeführt werden, sowie
(3) die partielle Hydrolyse eines Poly-2-oxazolins mit N-substituierten Einheiten unter Erhalt eines Copolymers mit unsubstituierten Ethylenimin-Einheiten mit anschließender Substitution bzw. Funktionalisierung von solchen Ethylenimin-Einheiten, insbesondere durch Substitution mit ungesättigten Gruppen.

<u>Verfahren vom Typ (1)</u>

Kationische ringöffnende Polymerisation

Ein typisches erfindungsgemäßes Verfahren zur Herstellung von N-substituierten Homopolymeren besteht in der kationischen ringöffnenden Polymerisation eines 2-substituierten 2-Oxazolins M nach folgender Reaktionsgleichung:

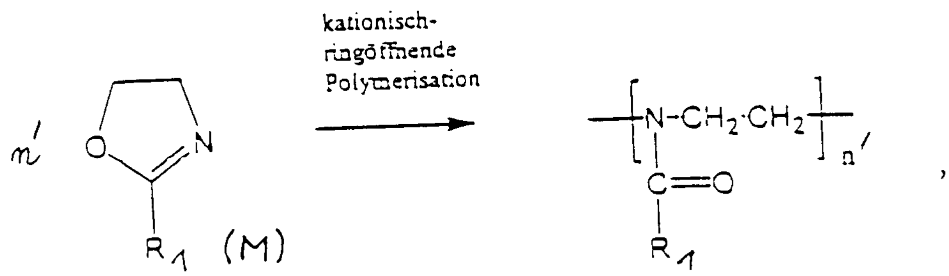


wobei $R_1$ wie oben definiert ist und
n' den mittleren Polymerisationsgrad bedeutet, der vorzugsweise im Bereich von 5 bis 500 liegt.

Die ringöffnende Polymerisation wird durch einen kationischen Initiator katalysiert, vorteilhaft durch einen mindestens bifunktionellen Initiator.

In analoger Weise werden ungesättigte Homopolymere durch ringöffnende Polymerisation eines 2-substituierten 2-Oxazolins N nach folgender Reaktionsgleichung erhalten:

Dabei bedeuten

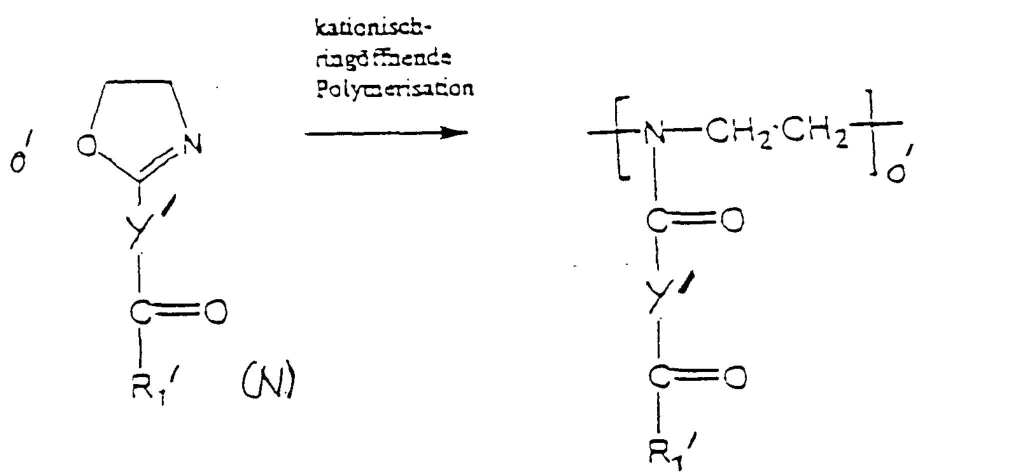


$R_1'$ dasselbe wie oben,
Y' eine Einfachbindung

oder

eine Gruppe der Formel $\{ CH_2 \}_p O$ —

mit p = 2 bis 7, die mit ihrem C-Ende am 2-Oxazolinring bzw. an der am N-Atom gebundenen CO-Gruppe gebunden ist,

und

o' den mittleren Polymerisationsgrad, der vorzugsweise im Bereich von 5 bis 500 liegt.

Auch hierbei wird ein kationischer Initiator verwendet.

Entsprechende Copolymere lassen sich durch kationische Copolymerisation eines oder mehrerer 2-substituierter 2-Oxazoline M mit einem oder mehreren 2-substituierten 2-Oxazolinen N herstellen.

Geeignete bifunktionelle Initiatoren vom Typ X-Z-X wurden bereits oben erläutert. Grundsätzlich können auch multifunktionelle Initiatoren wie etwa trifunktionelle oder höherfunktionelle Initiatoren, beispielsweise gesätttigte oder ungesättigte Trihalogenverbindungen oder Tetrahalogenverbindungen, eingesetzt werden.

Geeignete Lösungsmittel hierfür sind polare aprotische organische Lösungsmittel, wie Chloroform, Nitrobenzol und Acetonitril.

Im folgenden wird die kationische ringöffnende Polymerisation in Bezug auf die Verwendung eines bifunktionellen Initiators X-Z-X und des Monomers M erläutert. Wie bereits ausgeführt, ist trans-1,4-Dibrom-2-buten als Initiator günstig geeignet.

Bei der kationisch initiierten Polymerisation wird zunächst ein lebendes Polymer gebildet:

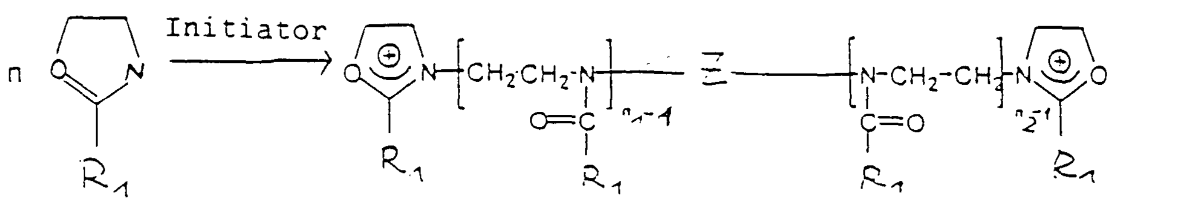


(M)

,

dessen Kettenenden noch aktiv sind. Dieses lebende Polymer wird anschließend mit einem nucleophilen Terminationsreagens, wie etwa Acrylsäure bzw. einer Alkylacrylsäure der Formel $H_2C = CH(R_a)\text{-}COOH$, funktionalisiert, wobei eine Base wie Triethylamin als Säureakzeptor eingesetzt wird. Diese Termination führt zu folgendem Polymer der Formel III, das bereits oben erläutert wurde:

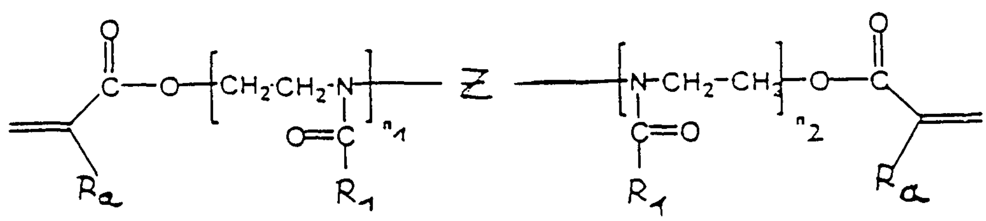

(III).

Wenn Gemische verschiedener 2-substituierter 2-Oxazoline eingesetzt werden, resultieren entsprechende Copolymere. Ein weiteres geeignetes Terminationsreagens ist z.B. Diallylamin.

Die geschilderte Verfahrensweise nach Typ (1) ist besonders vorteilhaft, da sie es erlaubt, ungesättigte Gruppen bei der Termination in das Polymermolekül einzuführen, so dass kein zusätzlicher Modifizierungsschritt erforderlich ist.

Die ungesättigten Endgruppen und gegebenenfalls auch eine oder mehrere Initiatorgruppen Z sind radikalisch vernetzbar.

Die Polydispersitätsindices dieser Polymeren liegen je nach Kettenlänge zwischen 1,1 und 1,25.

Verfahren vom Typ (2)

Polymeranaloge Umsetzung von Polyethyleniminen

Diese Verfahrensweise beruht auf der N-Substitution von Polyethyleniminen der allgemeinen Formel

$$-\!\!\left[ NH\text{-}CH_2\text{-}CH_2 \right]\!\!-_{n'}$$

mit n' wie oben definiert

durch Modifizierungsreagentien.

Solche Modifizierungsreagentien sind insbesondere Verbindungen der Formeln

$$R_1\text{-CO-X}$$

und

$$R_1'\text{-CO-Y-X,}$$

wobei X Halogen, insbesondere Cl, bedeutet.

Die Reaktion wird in Gegenwart eines Säureakzeptors durchgeführt, z.B. unter Verwendung von Pyridin. Die Umsetzung kann in organischen Lösungsmitteln durchgeführt werden, zum Beispiel in Methylenchlorid.

Durch geeignete Wahl der Modifizierungsreagentien lässt sich beispielsweise die Hydrophilie des Polymers einstellen.

Ein wichtiger Gesichtspunkt bei dieser Verfahrensweise ist die Möglichkeit, erheblich mehr ungesättigte und vernetzbare Gruppen in die Polymermoleküle einzuführen, als dies bei der Termination von kationisch erhaltenen lebenden Polymeren der Fall ist. Damit lassen sich physikalische Eigenschaften, die nach der Vernetzung resultieren, wie etwa das Quellvermögen, innerhalb weiterer Grenzen einstellen.

Dem Fachmann ist klar, dass die N-Substitution von Polyethyleniminen nicht nur mit den oben angeführten Halogeniden bzw. Säurehalogeniden möglich ist, sondern auch durch Einsatz entsprechender Säureanhydride oder unter Verwendung von Dicyclohexylcarbodiimid (DCC).

Wenn bei der Modifizierung nicht sämtliche Ethylenimin-Einheiten der Polymermoleküle am N-Atom substituiert werden, verbleiben entsprechend noch unsubstituierte Einheiten, so dass ein entsprechendes Copolymer vorliegt.

Die polymeranaloge Modifizierung von Polyethyleniminen kann auch unter Verwendung von mehreren Modifizierungsreagentien durchgeführt werden, wobei dann entsprechende ternäre oder höhere Copolymere resultieren.

Die oben erläuterte polymeranaloge Umsetzung führt zu einer statistischen oder quasistatistischen Verteilung der eingeführten Gruppen.

Verfahren vom Typ (3)

Partielle Hydrolyse eines Poly-2-oxazolins mit N-substituierten Einheiten und anschließende Funktionalisierung

Die Verfahrensweise des Typs (3) unterscheidet sich vom Verfahren nach Typ (2) nur darin, dass als Ausgangsmaterial zur polymeranalogen Modifizierung nicht ein Polyethylenimin eingesetzt wird, sondern ein Polymer, das in seinen Ketten Ethylenimin-Einheiten aufweist, aber nicht ausschließlich aus solchen Einheiten besteht.

Demgemäß wird von einem N-substituierten Polyethylenimin (N-substituierten Poly-2-oxazolin) ausgegangen, das vorteilhaft die Formel

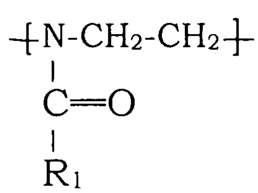

mit $R_1$ wie oben definiert besitzt. Dieses Ausgangspolymer kann in beliebiger Weise hergestellt sein, zum Beispiel nach einem Verfahren des Typs (1).

Ein Teil der N-Substituenten -CO-$R_1$ wird durch basische Hydrolyse abgespalten, so dass unsubstituierte Ethylenimin-Einheiten entstehen.

Der Hydrolysegrad kann über die Reaktionsbedingungen bei der Hydrolyse und deren Dauer eingestellt werden.

Ein Teil oder sämtliche entstandenen Ethylenimin-Einheiten werden dann in gleicher Weise wie beim Verfahren nach Typ (2) polymeranalog modifiziert bzw. funktionalisiert.

Die vollständige Modifizierung sämtlicher Ethylenimin-Einheiten führt dann im Ergebnis zu binären Copolymeren, während die partielle Modifizierung mit einem Modifizierungsmittel entsprechend zu ternären Copolymeren führt.

Im Rahmen der Erfindung können auch andere Reagentien zur Endgruppenerzeugung als die oben angeführten eingesetzt werden, da allgemein alle Verbindungen verwendbar sind, die mit dem lebenden Polymer unter Bildung von Endgruppen reagieren, die vorzugsweise mit ungesättigten, vernetzbaren Gruppen funktionalisiert sind.

Weitere geeignete Endgruppen sind beispielsweise Endgruppen der Formel

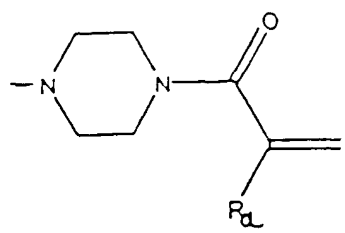

mit $R_a$ wie oben definiert.

Diese Gruppen können auf zwei Arten in das Polymer eingeführt werden:

- Umsetzung eines mit Piperazin terminierten N-substituierten Poly-2-oxazolins in einer polymeranalogen Umsetzung mit einem Acrylsäure- oder einem Alkylacrylsäurehalogenid oder
- Herstellung eines Terminationsreagens aus Piperazin und einem Acrylsäure- oder einem Alkylacrylsäurehalogenid und
- Terminierung eines lebenden 2-substituierten Poly-2-oxazolins mit dem erhaltenen Terminierungsreagens.

Selbstverständlich können im Rahmen der Erfindung auch andere bifunktionelle Amine und andere polymerisierbare Gruppen eingesetzt werden.

Ein solches weiteres Beispiel sind Endgruppen aus Piperazin und Glycidyl(alkyl)acrylsäureestern, die nach folgender Gleichung erhältlich sind:

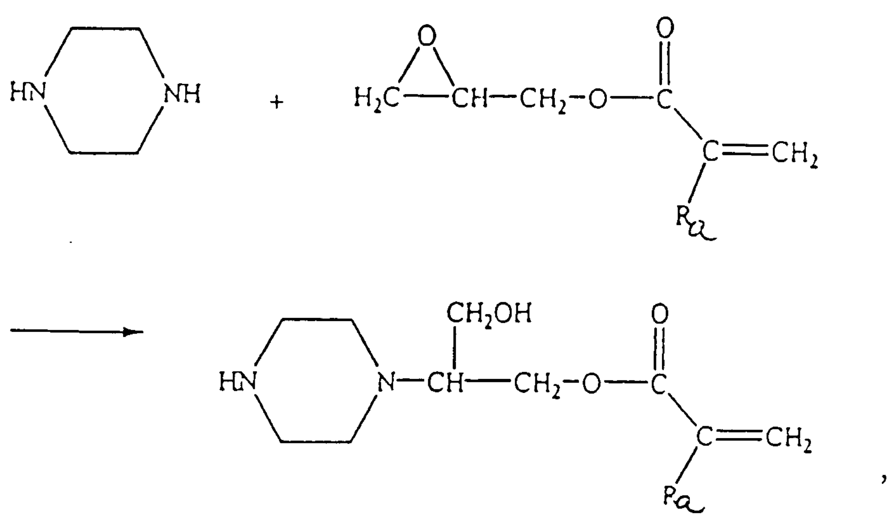

wobei $R_a$ die oben definierte Bedeutung besitzt.

Die mittleren Molekulargewichte (Gewichtsmittel $M_w$) der unvernetzten erfindungsgemäßen Polymeren liegen vorteilhaft im Bereich von etwa 500 bis etwa 100.000, besonders, wenn die Polymeren aus oder unter Verwendung von Polyethylenimin erzeugt sind.

Bei Erzeugung durch kationische ringöffnende Polymerisation von 2-substituierten 2-Oxazolinen liegen die mittleren Molekulargewichte $\overline{M}_w$ vorteilhaft im Bereich von etwa 500 bis etwa 20.000 und insbesondere im Bereich von etwa 500 bis etwa 5000.

Die nach den verschiedenen erläuterten Verfahren erhältlichen Homopolymeren und Copolymeren, die durch Endgruppen, Initiatorreste Z und/oder durch N-Substitution ungesättigte Gruppen enthalten, können in an sich bekannter Weise radikalisch vernetzt werden, vorteilhaft thermisch oder photochemisch durch UV-Bestrahlung in Gegenwart eines radikalischen Initiators. Auch eine strahleninduzierte Vernetzung unter Anwendung von Röntgen- oder Gammastrahlung ist möglich. Dem Fachmann sind hierbei verwendbare Sensibilisatoren, z.B. zur photochemischen Vernetzung, geläufig.

Radikalische Initiatoren sind dem Fachmann ebenfalls geläufig. Die Vernetzungsgeschwindigkeit und die Vernetzungsdichte können durch geeignete Wahl eines Initiators eingestellt werden. Beispiele für typische geeignete Initiatoren sind Benzoylperoxid, AIBN, 2,2'-Azobis[2-(2-imidazolin-2-yl)-propan]-dihydrochlorid, Azobis(2-amidinopropan)-dihydrochlorid (ABAH) und/oder Azobis(cyanopentansäure).

Die Vernetzung kann in einem Lösungsmittel durchgeführt werden. Die erfindungsgemäßen unvernetzten Polymeren lösen sich je nach Molekülmasse mehr oder weniger leicht in Wasser, Aceton oder Alkoholen wie Ethanol.

Die durch Vernetzung erhältlichen Polymeren stellen hydrophile, quellfähige Hydrogele mit hohem Absorptionsvermögen für Wasser und wässerige Systeme dar. Sie eignen sich daher günstig als Sorbentien und Flüssigkeitsabsorber für die verschiedensten Zwecke, besonders als Absorber zur Herstellung von oder in Klärmitteln für Flüssigkeiten, insbesondere in der Nahrungs- und Genussmittelindustrie und der chemischen Industrie, Verbandmaterialien, Hygieneartikeln, insbesondere Babywindeln, Damenbinden, Slipeinlagen, Tampons, Inkontinenzprodukten, Pflanzensubstraten und Verpakkungen und Lagerschalen für flüssigkeitsabsondernde Nahrungsmittel.

Von besonderer Bedeutung ist im Rahmen der Erfindung das Absorptionsvermögen für wässerige Flüssigkeiten, insbesondere Körperflüssigkeiten wie Blut und Sekrete, und Körperausscheidungen, wie Urin und Kot.

Das hohe Absorptionsvermögen der erfindungsgemäßen Polymeren ist mit dem Absorptionsvermögen der herkömmlichen sogenannten Superabsorber vergleichbar.

Ein besonders vorteilhafter Aspekt der Erfindung ist darin zu sehen, dass die unvernetzten oder nur gering vorvernetzten Polymeren in flüssigem Zustand, bevorzugt in Lösung in einem Lösungsmittel, auf ein Substrat aufgebracht oder darin eingebracht werden und dann auf oder im Substrat vernetzt werden können. Besonders geeignete Substrate oder Träger sind Vliese, die auch hydrophob sein können, und andere Flächengebilde, wie sie etwa für Slipeinlagen oder Babywindeln Verwendung finden.

Von besonderem Vorteil ist die Möglichkeit, die erfindungsgemäßen Absorber-Polymeren bereits während der Fertigung des Trägers, z.B. bereits während der Vliesfertigung, in einem kontinuierlichen Prozess aufzubringen und danach das Polymer zu vernetzen. Dadurch wird eine besonders innige Verbindung des Polymers mit dem Träger erzielt. Das Aufbringen oder die Imprägnierung eines Trägers können nach beliebigen Verfahren erfolgen, vorteilhaft durch Aufsprühen, Tauchen, Gießen oder Drucken. Damit sind polymerbeschichtete oder polymerimprägnierte Träger und damit Absorber und Saugkörper in beliebiger Gestalt und Größe herstellbar.

Im folgenden wird der Teil des Erfindungskonzepts erläutert, der sich auf die Verwendung der erfindungsgemäßen Polymeren als und in Sorbentien und Absorbern und die Herstellung solcher Artikel bezieht.

Diesem Gegenstand liegt die Aufgabe zugrunde, neuartige hydrophile Sorbentien und Absorber für Wasser und wässerige Flüssigkeiten und ihre Verwendung in entsprechenden Artikeln anzugeben, die hohe Quellfähigkeit und hohe Aufnahmekapazität besitzen, wobei eine gleichmäßige Flüssigkeitsverteilung in Absorbern erzielbar sein soll. Ferner sollen Verfahren zur Herstellung der Sorbentien und Absorber sowie entsprechender Artikel angegeben werden.

Die Aufgabe wird gemäß den entsprechenden unabhängigen Ansprüchen gelöst. Die abhängigen Ansprüche betreffen vorteilhafte Ausführungsformen dieses Erfindungskonzepts.

Die erfindungsgemäßen Homopolymeren und Copolymeren eignen sich als Sorbentien und Absorber und zur Herstellung von Sorbentien und Absorbern zur Absorption von Wasser und wässerigen Flüssigkeiten, insbesondere von Körperflüssigkeiten wie Blut sowie von Körperausscheidungen, wie Menstruationsblut, Urin, Sekreten und Kot.

Die erfindungsgemäßen Polymeren lassen sich besonders vorteilhaft als oder in Klärmitteln für Flüssigkeiten, besonders in der Nahrungsmittel- und Getränkeindustrie und der chemischen Industrie, für absorbierende Einwegartikel, wie sie im Hygienebereich eingesetzt werden, also zum Beispiel für Babywindeln, Damenbinden, Slipeinlagen, Erwachsenen-Inkontinenzhilfen, Tampons und dergleichen, für Verbandmaterialien, Verpackungen, Lagerschalen für flüssigkeitsabsondernde Nahrungsmittel, Pflanzensubstrate, besonders Pflanzmatten, und allgemein alle Anwendungen einsetzen, bei denen es auf eine hohe Flüssigkeitsaufnahme und gleichmäßige Flüssigkeitsspeicherung ankommt.

Die erfindungsgemäßen Flüssigkeiten absorbierenden Artikel, die insbesondere Flächengebilde darstellen, weisen ein Substrat oder einen Träger auf, auf dem bzw. in dem ein oder mehrere Polymere gemäß der Erfindung,

die vorzugsweise vernetzt sind, vorliegen.

Die Verbindung zwischen dem Substrat und dem Polymer wird vorteilhaft so erzeugt, dass das Substrat mit dem unvernetzten oder nur gering vernetzten Polymer, gegebenenfalls in einem Lösungsmittel, befeuchtet bzw. getränkt wird, worauf das Polymer vernetzt bzw. abschließend vernetzt wird. Dadurch wird eine besonders innige Verbindung mit dem Substrat erzielt.

Als Substrate kommen beliebige Materialien und Artikel in Frage, vorteilhaft Faserstoffe, Vliese, Textilgewebe oder Granulate von Trägermaterialien.

Herkömmliche Superabsorber bestehen aus einem Material auf der Basis von Polyacrylsäure und sind fast ausschließlich in salzartiger Pulverform erhältlich. Sie besitzen zwar ein sehr hohes Quellvermögen in Wasser, verlieren aber bis zu 80 % ihres Quellvermögens in Lösungen mit einem Salzgehalt (Urin, Schweiß, Blut). Außerdem sind herkömmliche Superabsorber spröde, so dass sie sich gerade bei der Herstellung von absorbierenden Hygieneartikeln nur schwer verarbeiten lassen.

Konventionelle Superabsorber werden als Partikel auf die Substrate, wie fluff-pulp-ähnliche Strukturen und insbesondere Vliese, aufgestreut oder aufdispergiert und sind entsprechend nur in geringem Maße am Substrat verankert und bleiben ungeordnet und lose. Aufgrund der raschen Quellung bei Flüssigkeitskontakt tritt ferner der sog. Gel-blocking-Effekt auf, der bewirkt, dass das Absorbergel das tiefere Eindringen der Flüssigkeit aufgrund der raschen Oberflächenquellung behindert und sich Quellungsgleichgewichte über die gesamte Absorberdicke nur sehr langsam einstellen. Außerdem wird die Flüssigkeit in herkömmlichen Superabsorbern aufgrund der partikelartigen Struktur nicht gleichmäßig verteilt.

Im Gegensatz dazu sind die erfindungsgemäßen vernetzten Polymeren und entsprechende Absorber in der Lage, hohe Flüssigkeitsmengen rasch über die gesamte Dicke zu binden. Sie können in Form eines unvernetzten oder nur gering vernetzten Polymers auf ein Substrat aufgebracht und darauf bzw. darin vernetzt werden. Dies führt zu einer starken Verbindung mit dem Substrat und gleichzeitig zu einer sehr gleichmäßigen Polymerverteilung. Da die erfindungsgemäßen vernetzten Polymeren im Vergleich zu herkömmlichen Superabsorbern langsamer quellen, werden Gel-blocking-Effekte und damit eine Rücknässung praktisch vollständig vermieden. Erfindungsgemäße Absorber sind befähigt, Flüssigkeit auch unter Druckbelastung ausreichend zu binden.

Typischerweise besitzt ein erfindungsgemäßer absorbierender Artikel, wie er etwa für Damenbinden und Slipeinlagen verwendet wird, folgenden prinzipiellen Aufbau:

- eine flüssigkeitsundurchlässige Grundschicht, die der Bekleidung des Trägers zugewandt ist,
- eine luft- und flüssigkeitsdurchlässige Deckschicht, die dem Körper des Trägers zugewandt ist, und
- eine zwischen der Grundschicht und der Deckschicht vorgesehene flüssigkeitsabsorbierende Saugschicht, die einen Vliesstoff niederer Dichte enthält,

und ist dadurch gekennzeichnet, dass der Vliesstoff ein oder mehrere vernetzte Polymere gemäß der Erfindung enthält, die durch Imprägnierung mit unvernetztem oder vorvernetztem Polymer und anschließende Vernetzung am bzw. im Vliesstoff gebunden sind.

Zwischen der Deckschicht und der Saugschicht kann ferner eine Verteilschicht angeordnet sein, die eine gleichmäßige Verteilung der zu absorbierenden Flüssigkeit im Absorber fördert. Dieser flüssigkeitsabsorbierende Artikel funktioniert, vereinfacht dargestellt, in der Weise, dass die vom Körper abgegebenen Flüssigkeiten die köperseitige, flüssigkeitsdurchlässige Schicht durchdringen und, gegebenenfalls nach Verteilung durch eine Verteilschicht, in der flüssigkeitsabsorbierenden Saugschicht aufgenommen und festgehalten werden. Die rückseitige, flüssigkeitsundurchlässige Grundschicht verhindert, dass Teile der von der Saugschicht aufgenommenen Flüssigkeit den absorbierenden Artikel in Richtung Träger verlassen und dessen Kleidung verschmutzen.

Die Deckschicht besteht bevorzugt aus einem Vliesstoff, insbesondere einem hydrophoben perforierten Vliesstoff, jedoch können auch perforierte Folien Verwendung finden, die zusätzlich mit einem Vliesstoff verbunden sein können. Eine geeignete Folien-Deckschicht ist z.B. in WO 96/26697 beschrieben. Perforierte Vliesstoffe sind etwa in EP 0 164 740 beschrieben.

Der Träger, besonders ein Vliesstoff, kann hydrophil oder hydrophob sein. Sehr günstige Quelleigenschaften ergeben sich mit hydrophoben Vliesen als Träger.

Nach einer vorteilhaften Ausführungsform ist die Saugschicht mehrschichtig ausgebildet und besteht aus einer ersten Kernschicht aus einem hydrophilen synthetischen Mikrofaservlies, die von einem Vliesstoff niederer Dichte umhüllt ist, und einer zweiten Kernschicht aus einem hydrophilen synthetischen Mikrofaservlies, die mit einer weiteren Kernschicht aus hoch wasserspeicherndem Material in Kontakt ist, wobei eine oder mehrere Schichten der Saugschicht ein oder mehrere Polymere gemäß der Erfindung enthalten. Dieser mehrschichtige Aufbau der Saugschicht ist per se in DE 199 15 716 beschrieben.

Die weitere Kernschicht kann vorteilhaft neben erfindungsgemäßen Polymeren Cellulosefasern und/oder superabsorbierende Partikel und/oder superabsorbierende Fasern enthalten; ferner können auch Mikrofasern enthalten

sein.

Wenn das Material der Saugschicht hydrophil ist und beispielsweise einen Vliesstoff darstellt, handelt es sich vorteilhaft um einen hydrophilierten Polypropylen-Meltblown-Vliesstoff.

Es ist klar, dass die erfindungsgemäßen absorbierenden Artikel mit weiteren Teilen ausgestattet sein können, z.B. mit Verschlüssen, Klebestreifen, elastischen Bändern, Beinabschlüssen, Haftsystemen, etc..

Die für die verschiedenen Schichten verwendbaren Vliesstoffe sind dem Fachmann als solche geläufig (vgl. die Monographie "Vliesstoffe", Georg Thieme Verlag, Stuttgart, New York). Beispiele für geeignete Vliesstoffe sind kardierte, thermisch verfestigte oder durch Bindemittel verfestigte Vliesstoffe, ferner Spinnvliesstoffe, Meltblown-Vliesstoffe und Kombinationen solcher Vliesstoffe sowie wasserstrahlverfestigte Vliesstoffe.

Die flüssigkeitsdurchlässige Deckschicht hat üblicherweise eine Flächenmasse von 8 bis 100 g/$m^2$ und bevorzugt 12 bis 50 g/$m^2$.

Die Vliesstoffe können insbesondere aus Polyethylen, Polyester, Polyamid, Polyarylnitril, Viskose oder Naturfasern oder aus Mischungen solcher Rohstoffe bestehen. Günstig geeignete Naturfasern sind Cellulose-Pulp, Hanf, Jute, Flachs, Sisal, Baumwolle, Kenaf und Kapok.

Bevorzugt bestehen die für die flüssigkeitsdurchlässige Deckschicht verwendeten Rohstoffe aus Polyolefinen wie Polyethylen oder Polypropylen. Ferner können auch Bikomponentenfasern verwendet werden, deren Kern beispielsweise aus Polypropylen und deren Mantel beispielsweise aus Polyethylen besteht.

Die Grundschicht ist bevorzugt ein wasserdichtes Material, beispielsweise eine Kunststofffolie, etwa aus Polyethylen, die mit einem Vliesstoff kaschiert sein kann, um textilartige haptische Eigenschaften zu erzielen.

Die Grundschicht ist vorteilhaft wasserdicht, aber wasserdampfdurchlässig ausgeführt. Sie kann beispielsweise gemäß WO 97/16148 aus mindestens einer Spinnvlieslage und zwei Meltblown-Lagen bestehen.

Die Saugschicht bzw. eine oder mehrere Schichten, aus denen die Saugschicht zusammengesetzt ist, kann neben dem erfindungsgemäßen Absorberpolymer noch einen herkömmlichen Superabsorber, etwa auf Acrylsäurebasis enthalten, der in Pulverform vorliegt, wobei die Partikel des Superabsorbers vorzugsweise durch das erfindungsgemäße vernetzte Polymer am Substrat, vorteilhaft einem Vlies, gebunden sind.

Das erfindungsgemäße Verfahren zur Herstellung der absorbierenden Artikel umfasst folgende Schritte:

(1) Aufbringen einer Lösung eines oder mehrerer unvernetzter oder vorvernetzter Polymerer gemäß der Erfindung sowie eines radikalischen Initiators in einem Lösungsmittel auf ein Substrat, insbesondere eine für die Saugschicht vorgesehene Schicht,
(2) Verdampfen des Lösungsmittels und
(3) Vernetzung oder Nachvernetzung des Polymers auf bzw. im Substrat durch thermische und/oder photochemische Vernetzung, insbesondere durch UV-Bestrahlung, sowie gegebenenfalls
(4) Nachreaktion zur Vervollständigung der Vernetzung.

Als Lösungsmittel wird vorteilhaft Wasser oder ein polares organisches Lösungsmittel verwendet. Beispiele hierfür sind Aceton sowie aliphatische Alkohole, wie Ethanol.

Als Initiatoren eignen sich prinzipiell alle radikalischen Initiatoren geeigneter Löslichkeit. Die Auswahl des Initiators und der Initiatorkonzentration ist dem Fachmann geläufig. Durch geeignete Wahl des Initiators und seiner Konzentration können die Vernetzungsgeschwindigkeit und die Vernetzungsdichte eingestellt werden.

Beispiele für geeignete Initiatoren sind Benzoylperoxid, AIBN, 2,2'-Azobis[2-(2-imidazolin-2-yl)-propan]-dihydrochlorid, Azobis(2-amidinopropan)-dihydrochlorid (ABAH) und Azobis(cyanopentansäure). Es können auch Kombinationen mehrerer Initiatoren eingesetzt werden.

Die Erfindung wird im folgenden anhand von Beispielen unter Bezug auf die Zeichnungen näher erläutert; es zeigen:

Fig. 1 und 2 einen erfindungsgemäßen flüssigkeitsabsorbierenden Artikel in Form einer Windel oder Slipeinlage im Querschnitt,

Fig. 3 eine rasterelektronenmikroskopische Aufnahme einer mit einem erfindungsgemäßen Polymer belegten Vliesfaser,

Fig. 4 eine rasterelektronenmikroskopische Aufnahme einer Vliesfaser eines erfindungsgemäßen Komposit-Absorbers, bei dem ein herkömmlicher Superabsorber durch ein erfindungsgemäßes Polymer auf den Vliesfasern gebunden ist und

Fig. 5 eine schematische Darstellung einer Vorrichtung zur Durchführung des erfindungsgemäßen Verfahrens in Ansicht von oben auf die Deckschicht, teilweise geschnitten.

Der in den Figuren 1 und 2 dargestellte Artikel 1 besitzt den bereits oben beschriebenen prinzipiellen Aufbau. Zwischen der Deckschicht 2 und der Grundschicht 3, die im gezeigten Beispiel am Rand miteinander verbunden sind,

ist eine Saugschicht 5 vorgesehen, die ein erfindungsgemäßes vernetztes Polymer als Absorber oder zumindest als Coabsorber enthält. Der gezeigte Artikel weist ferner eine im Rahmen der Erfindung fakultative Verteilschicht 4 auf, die zwischen der Deckschicht 2 und der Saugschicht 5 angeordnet ist.

**Beispiel 1**

Methacrylsäure- und Acrylsäure-terminierte Poly(2-methyl-2-oxazoline) (Verfahren Typ (1))

Ein fest verschraubbarer Schlenk-Kolben mit $N_2$-Anschluß, vorzugsweise mit PTFE-Hahn, und Magnetrührstab wird dreimal ausgeheizt und jeweils mit getrocknetem $N_2$ begast. Im Stickstoffgegenstrom werden 20 bis 30 ml Acetonitril und 2-Methyl-2-oxazolin (ca. 25 %ig in Acetonitril) vorgelegt. Bei Raumtemperatur wird 1,4-Dibrom-2-buten ebenfalls im $N_2$-Gegenstrom zugegeben. Das Reaktionsgemisch wird bei 80 °C 12 bis 24 h gerührt. Anschließend wird die Polymerisation mit Methacrylsäure oder Acrylsäure abgebrochen (fünffacher Überschuß bezogen auf den Initiator) und bei 60 °C eine halbe Stunde gerührt. Danach wird eine zur Säure äquivalente Menge an Triethylamin oder DBU hinzugefügt und weitere 12 h gerührt.

Die Lösung wird dann am Rotationsverdampfer im Grobvakuum bis zur Trockne eingeengt. Der Rückstand wird mit 20 ml Chloroform aufgenommen und mit 2 bis 3 g ausgeheiztem Kaliumcarbonat 12 h gerührt. Die Lösung wird anschließend mit einer Spritze abgesaugt, durch ein Spritzenfilter (PTFE-Membran) filtriert und erneut im Vakuum bis zur Trockne eingeengt. Der Rückstand wird mit Chloroform oder Methylenchlorid aufgenommen und dreimal in Diethylether oder n-Hexan gefällt. Der gelblich-weiße Niederschlag wird abgesaugt und abschließend mehrere Tage im Feinvakuum getrocknet.

Acrylsäure-terminiert:

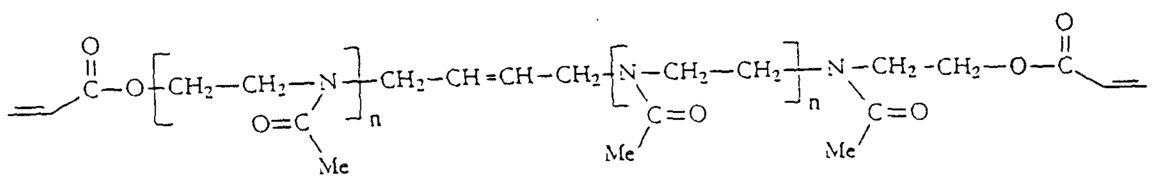

Methacrylsäure-terminiert:

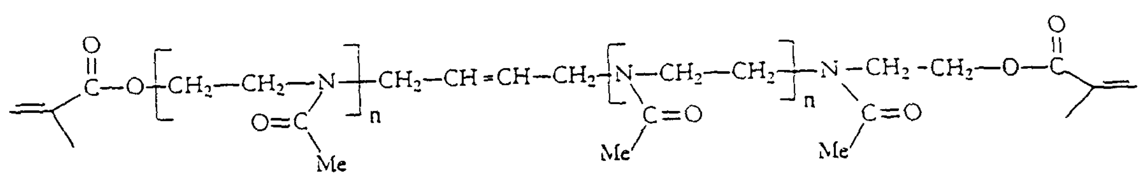

**Beispiel 2**

Modifizierung von partiell verseiftem Poly(2-methyl-2-oxazolin) (Verfahren Typ (3))

(a) Verseifung von Poly(2-methyl-2-oxazolin)

18,8 g eines mit Diallylamin terminierten Poly(2-methyl-2-oxazolins) mit einem Molmassen-Zahlenmittel von 2800 g/mol werden in 200 ml einer 1,3-molaren NaOH-Lösung 2 h auf 120 °C erhitzt. Die Lösung wird dann am Rotationsverdampfer zur Trockne eingeengt. Der aus Polymer, NaOH und Natriumacetat bestehende Feststoff wird 2 h in Methylenchlorid digeriert, wobei sich das teilverseifte Polymer löst. Der Niederschlag wird abfiltriert und noch zweimal wie oben beschrieben mit Methylenchlorid extrahiert.

Die organischen Phasen werden vereinigt und am Rotationsverdampfer vom Lösungsmittel befreit.

Das Polymer wird zum Abschluss zweimal aus Methylenchlorid in Ether gefällt, abfiltriert und im Vakuum getrocknet.

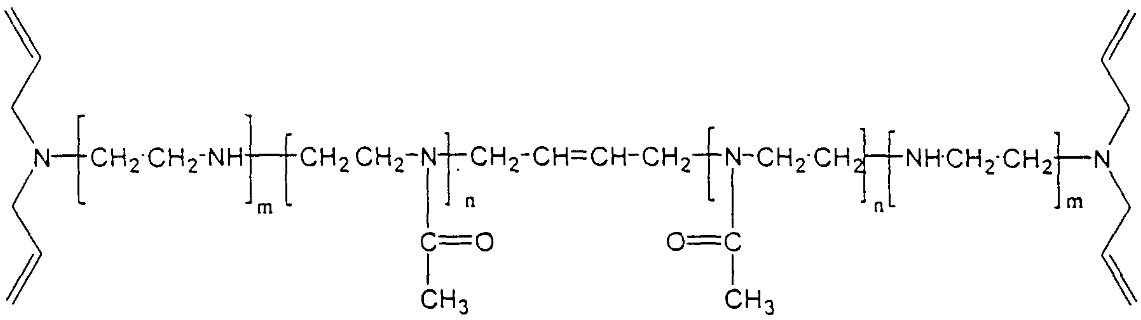

Ausbeute: 11,95 g.

(b) Poly(2-methyl-2-oxazolin)-co-poly(N-acryloylethylenimin)

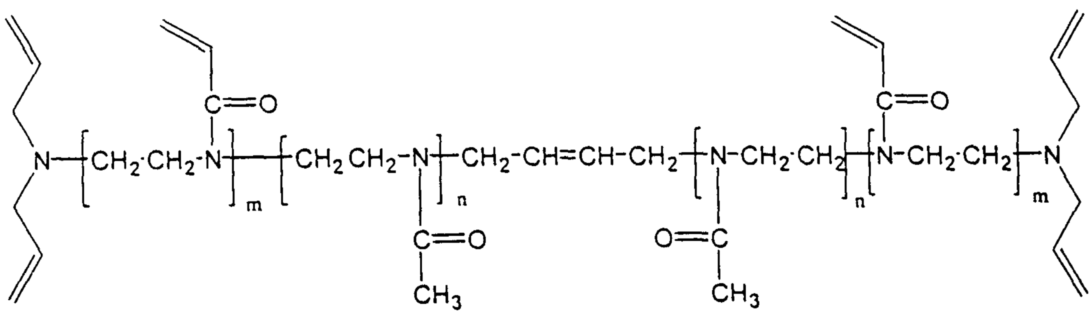

1. Methode:

3,50 g des im ersten Reaktionsschritt (a) gewonnenen partiell verseiften Poly(2-methyl-2-oxazolins) werden in 30 ml trockenem Methylenchlorid gelöst und mit 1,03 g Pyridin (13 mmol) versetzt. Zu dieser Lösung werden bei 0 °C eine Lösung von 1,49 g Acryloylchlorid (16 mmol) in 16 ml Methylenchlorid getropft. Die Lösung wird bei Raumtemperatur 12 bis 24 h gerührt.

Während der Reaktion fällt ein weißer Niederschlag (Pyridinium-Hydrochlorid) aus. Nach der Reaktionszeit wird die Suspension filtriert und am Rotationsverdampfer vom Lösungsmittel befreit. Der erhaltene Feststoff wird zweimal aus Methylenchlorid in Ether gefällt und im Vakuum getrocknet.

Ausbeute: > 80 % (bestimmt aus NMR).

2. Methode:

In einer Stickstoffatomosphäre wird eine Lösung aus 370 mg Acrylsäure (5,1 mmol) in 10 ml trockenem Dichlormethan mit 1,80 g N,N'-Dicyclohexylcarbodiimid (DCC) (8,7 mmol) bei 0 °C versetzt. Die Lösung wird 30 min gerührt, wobei nach dem Auflösen des DCC ein Niederschlag des DC-Harnstoffs beobachtet wird.

Anschließend wird eine ca. 20 %ige Lösung des partiell verseiften Poly(2-methyl-oxazolins) in Dichlormethan hinzugefügt. Das Verhältnis von Säure zu -NH-Gruppen im Polymer beträgt etwa 2.

Das Reaktionsgemisch wird dann 3 Tage bei Raumtemperatur gerührt. Der Niederschlag wird filtriert und die Lösung mit dem funktionalisierten Polymer am Rotationsverdampfer vom Lösungsmittel befreit.

Zur Reinigung wird das Polymer dreimal in Chloroform gelöst und in Diethylether gefällt.

Ausbeute: > 90 %, bezogen auf die Polyethylenimin-Einheiten.

## Beispiel 3

Vernetzung von erfindungsgemäßen Polymeren

Allgemeines Vorgehen:

Für eine erfolgreiche Vernetzung ist es sehr wichtig, dass das zu vernetzende Polymer und der Initiator sehr gut miteinander vermischt werden. Im besten Fall sind der Initiator und das Polymer ineinander löslich. Im anderen Fall erreicht man die bestmögliche Verteilung, indem das Polymer und der Initiator im gleichen Lösungsmittel gelöst und die Lösungen vereinigt werden. Das Lösungsmittel wird dann entfernt und die Vernetzung je nach Art des Initiators durch UV-Licht oder Hitze gestartet. Wenn nicht anders angegeben, wurde mit einer bei photochemischer und thermischer Vernetzung üblichen Konzentration des Initiators von 5 Masse-%, bezogen auf das Polymer, gearbeitet.
Es können ungiftige Lösungsmittel wie Wasser, Aceton oder Ethanol verwendet werden, was besonders vorteilhaft ist, wenn entsprechende vernetzte Absorber in Hautkontakt kommen können, da das Vorliegen geringer Lösungsmittelspuren im Endprodukt nicht völlig auszuschließen ist.
Die Vernetzung erfolgt vorteilhaft nach Aufbringen des zu vernetzenden Polymers auf ein Substrat wie ein Vlies.

Thermische Vernetzung:

In einem 100 ml-Rundkolben werden eine bestimmte Menge des modifizierten Poly(2-methyl-2-oxazolins) und 3 bis 5 Masse-% Initiator (z.B. AIBN, Benzoylperoxid) zusammen in 30 ml Aceton gelöst. Das Lösungsmittel wird dann im Vakuum entfernt und der Sauerstoffgehalt durch wechselndes Evakuieren, Fluten mit Stickstoff und Ausheizen des Kolbens auf ein Minimum reduziert. Das Gemisch wird anschließend im Ölbad oder in einem Trockenschrank 30 min auf 80 °C erwärmt. Nach der Vernetzung werden eventuelle lösliche Anteile dreimal mit je 10 ml Ethanol extrahiert.

Photochemische Vernetzung in einem Vlies:

Da die Photoinitiatoren lichtempfindlich sind, werden alle Vorarbeiten in abgedunkelter Umgebung durchgeführt und die Zeit für die Probenvorbereitung auf ein Minimum verkürzt.
Wie bei der thermischen Vernetzung werden auch hier das zu vernetzende Polymer und der Initiator, in diesem Fall ein Photoinitiator, im gleichen Lösungsmittel gelöst und die Lösungen vereinigt. Um den Sauerstoffgehalt zu minimieren, wird 2 min ein Stickstoffstrom durch die Lösung geleitet.
Für das Aufbringen auf ein Vlies wird die Lösung mit einer Spritze punktuell auf das Vlies aufgetragen. Anschließend wird das Vlies ohne Zeitverzögerung in einen mit Stickstoff beschickten UV-Belichtungsapparat gebracht, worin es 10 min ohne Belichtung von Stickstoff umspült wird.
Die Belichtung erfolgt 90 s. Nach der Belichtungsphase wird das beschichtete Vlies weitere 5 min nachreagieren gelassen.
Die Polymeren weisen nach der Vernetzung ein gutes bis sehr gutes Quellvermögen in wässerigen Medien auf. Durch das Verhältnis der beiden Comonomeren ist der Grad der Vernetzung und somit auch das Quellvermögen in einem weiten Bereich einstellbar.
Die Polymeren besitzen hohe Flüssigkeitsaufnahme und sind praktisch frei von Gel-blocking-Effekten.
Die Netzbogenlänge, also der Abstand zwischen zwei Netzpunkten, liegt bei erfindungsgemäßen Polymeren in der Größenordnung von etwa 1300 $g \cdot mol^{-1}$, was im Mittel nur etwa 15 Monomereinheiten entspricht, während die Netzbogenlänge bei kommerziell erhältlichen Superabsorbern nach dem Stand der Technik im Bereich von einigen 100.000 $g \cdot mol^{-1}$ liegt.
Bei den erfindungsgemäßen vernetzten Polymeren handelt es sich ferner um elektrisch neutrale Hydrogele, während herkömmliche Superabsorber Carboxylgruppen in der Gelmatrix aufweisen, was die ausgeprägte Abhängigkeit des Quellvermögens von der Salzkonzentration oder der Ionenstärke in der wässerigen Lösung bei herkömmlichen Superabsorbern erklärt.
Fig. 3 zeigt eine rasterelektronenmikroskopische Aufnahme einer mit erfindungsgemäßem Absorber-Polymer belegten hydrophoben Faser.
In dem Kreisausschnitt ist zu erkennen, dass das Polymer die Faser sehr gleichmäßig umgibt.
Das unvernetzte Polymer war das Produkt von Beispiel 2 (b), in dessen Formel $m \approx 2$ und $n \approx 10$ waren. Das Material war praktisch vollständig vernetzt (löslicher Anteil etwa 2 %).
Im Rahmen dieses Beispiels wurde ferner ein Komposit-Absorber hergestellt, bei dem das erfindungsgemäße Polymer vor der Vernetzung mit einem herkömmlichen Superabsorber-Pulver kombiniert wurde, um im vernetzten Produkt eine Kombination der Eigenschaften beider Absorbermaterialien zu erzielen.

Dazu wurde ein herkömmliches Superabsorber-Pulver auf Acrylsäurebasis mit einer Lösung des erfindungsgemäßen Polymers und eines Photoinitiators vermischt und auf ein Vlies aufgetragen. Als Lösungsmittel kommen auch hierbei hinreichend polare Lösungsmittel wie niedere aliphatische Alkohole und deren Gemische in Frage.

Nach Verdampfen des Lösungsmittels wurde das Vlies wie oben beschrieben mit einer UV-Lampe bestrahlt. Alternativ kann der Absorber auch thermisch vernetzt werden, z.B. bei 80 bis 120 °C.

Die Superabsorber-Partikel werden durch das erfindungsgemäße Polymer wie durch einen Kleber an den Vliesfasern fixiert, so daß der gesamte Polymergehalt des Absorbers an den Fasern gebunden ist.

Fig. 4 zeigt eine rasterelektronenmikroskopische Aufnahme dieses Komposit-Absorbers. Der Kreisausschnitt läßt die Bindung eines Superabsorber-Partikels über das Polymer auf der Faser erkennen.

Das unvernetzte Polymer war das Produkt von Beispiel 2 (b), in dessen Formel $m \approx 2$ und $n \approx 10$ waren. Das Material war praktisch vollständig vernetzt (löslicher Anteil etwa 2 %).

Die so beschichteten Fasern zeichnen sich durch ein erhöhtes absolutes Quellvermögen und auch durch eine gesteigerte Quellungsgeschwindigkeit gegenüber dem herkömmlichen Absorber aus. Trotzdem bleiben die Fasern für eine Rollenlagerung flexibel genug.

**Beispiel 4**

Die Imprägnierung eines Substrats, besonders eines Vlieses, mit der Lösung eines erfindungsgemäßen Polymers und seine Vernetzung lassen sich in besonders vorteilhafter Weise kontinuierlich durchführen, wobei sich diese Schritte vorzugsweise unmittelbar an die Herstellung und Verfestigung des Vlieses anschließen. Dadurch resultiert ein einziger kontinuierlicher Prozess der Absorberherstellung, was technisch wie wirtschaftlich sehr vorteilhaft ist.

Eine Vorrichtung, mit der dieses integrierte Verfahren durchführbar ist, ist in Fig. 5 dargestellt.

Nach der Vliesfertigung 7 und der Verfestigung 8 des Vlieses, das beispielsweise, wie aus der Detailzeichnung in Fig. 3 hervorgeht, aus Zellstoff, Polypropylen und/oder Polyester besteht, wird aus einem Behälter 9 eine Lösung des zu vernetzenden Polymers und eines Initiators auf die Vliesbahn aufgebracht, z.B. durch Tauchen, Aufsprühen, Aufgießen, Drucken und andere Auftragverfahren. Das Lösungsmittel wird danach unter Erhitzen aus der Vliesbahn abgedampft, vorteilhaft in einem Heizbereich 10. Danach gelangt die mit Polymer imprägnierte Vliesbahn zu einer Vernetzungsstation 11, wo eine photochemische und/oder thermische Vernetzung vorteilhaft bei 40 bis 200 °C und insbesondere bei 60 bis 120 °C stattfindet. Im Nachreaktionsbereich 12 findet die Vervollständigung der Vernetzung statt, so daß ein nicht klebriges Absorbervlies resultiert. Das fertige Produkt wird dann zu einer Rolle aufgewickelt.

Die Imprägnierung, Vernetzung und Nachreaktion lassen sich dabei mit der bei der Vliesfertigung vorliegenden Bahngeschwindigkeit durchführen, so daß ein geschlossener kontinuierlicher Gesamtprozess realisiert werden kann.

Ab Molekulargewichten $\overline{M}_w$ von etwa 1500 sind die unvernetzten Polymeren hochviskos, bleiben aber in der Regel bis zu Molekulargewichten von etwa 2000 streichbar.

Bei Verwendung erfindungsgemäßer Polymerer zum Heissauftrag ohne Lösungsmittel können Molekulargewichte von bis zu etwa 15.000 gut eingesetzt werden.

Bei Verwendung eines Lösungsmittels, z.B. von Wasser, können die zum Auftragen vorgesehenen Polymeren praktisch beliebig hohe Molekulargewichte aufweisen.

## Patentansprüche

1. Homopolymere und Copolymere, die aus folgenden Einheiten A und/oder B bestehen oder diese Einheiten enthalten:

(A)

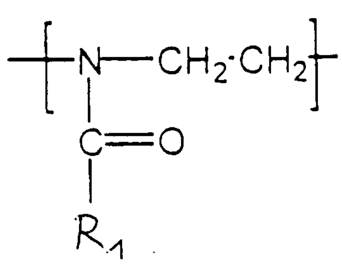

und/oder

(B) 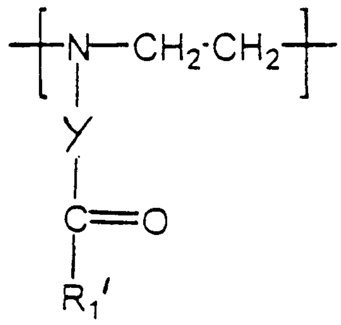 ,

wobei bedeuten:

$R_1$ Alkyl, insbesondere niederes Alkyl, oder eine hydrophile Gruppe,
$R_1'$ eine radikalisch vernetzbare ethylenisch ungesättigte Gruppe oder eine Gruppe, die eine radikalisch vernetzbare ethylenisch ungesättigte Gruppe aufweist,

und

Y eine Einfachbindung

oder
eine Gruppe der Formel $-CO-(CH_2)_p-O-$ oder $-(CH_2)_p-O-$ mit p = 2 bis 7, die jeweils mit dem C-Ende am N-Atom gebunden ist.

**2.** Homopolymere und Copolymere nach Anspruch 1, in deren Einheiten A die Gruppe $R_1$ bedeutet:

- $C_{1-6}$-Alkyl, insbesondere Methyl, Ethyl, Propyl oder Butyl, und/oder
- eine hydrophile Gruppe, die ausgewählt ist unter Hydroxyalkyl, insbesondere $C_{1-6}$-Hydroxyalkyl und vorzugsweise Hydroxymethyl, Hydroxyethyl und Hydroxypropyl $HO-CH_2CH_2-CH_2-$, Polyoxyethylengruppen

$$-[O-CH_2-CH_2]_b-$$

OH mit b im Bereich von 1 bis 20, die an der CO-Gruppe gebunden sind, und
Alkylgruppen, die eine unsubstituierte oder substituierte Ammoniumgruppe tragen, insbesondere Ammoniomethyl $R_3\overset{\oplus}{N}-CH_2-$, Ammonioethyl $R_3\overset{\oplus}{N}-CH_2-CH_2-$ und Ammoniopropyl $R_3\overset{\oplus}{N}-(CH_2)_3-$ mit R = H oder Alkyl, besonders niederes $C_{1-6}$-Alkyl.

**3.** Homopolymere und Copolymere nach Anspruch 1 oder 2, in deren Einheiten B die Gruppe $R_1'$ Vinyl $H_2C=CH-$, 1-Methylvinyl $H_2C=C(CH_3)-$, 1-Ethylvinyl $H_2C=C(C_2H_5)-$, Propenyl, Allyl oder eine von Zimtsäure

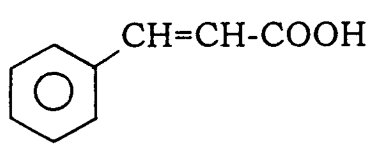

abgeleitete Gruppe bedeutet, die über eine Esterbindung an der Carboxylgruppe, an einem der C-Atome der ungesättigten Bindung oder am Phenylring gebunden ist.

**4.** Copolymere nach einem oder mehreren der Ansprüche 1 bis 3,
**dadurch gekennzeichnet, dass** sie zusätzlich zu den Einheiten A und/oder B Einheiten C der folgenden Formel

aufweisen:

(C) $-[NH-CH_2-CH_2]-$.

**5.** Copolymere nach einem oder mehreren der Ansprüche 1 bis 3 der Formel I,

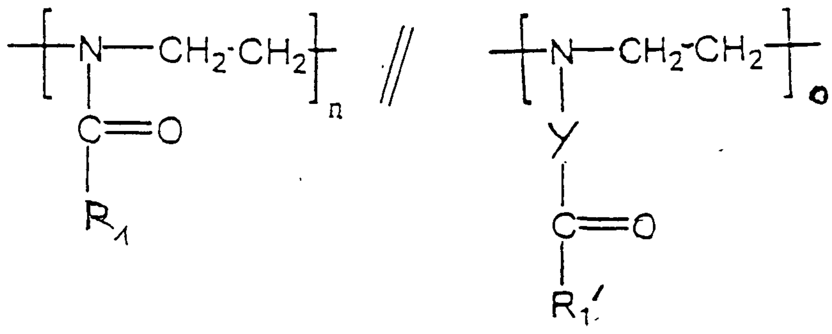

(A) (B) (I),

worin bedeuten:

| | |
|---|---|
| $R_1$, $R_1$' und Y | dasselbe wie in den Ansprüchen 1 bis 3, |
| n | 5 bis 300, vorzugsweise 15 bis 100, |

und

o 2 bis 50, vorzugsweise 3 bis 20,

wobei der Doppelstrich // bedeutet, dass die Einheiten A und B in den Polymerketten statistisch verteilt oder in Form von Blöcken beliebiger Abfolge vorliegen.

**6.** Copolymere nach einem oder mehreren der Ansprüche 1 bis 4 der Formel II,

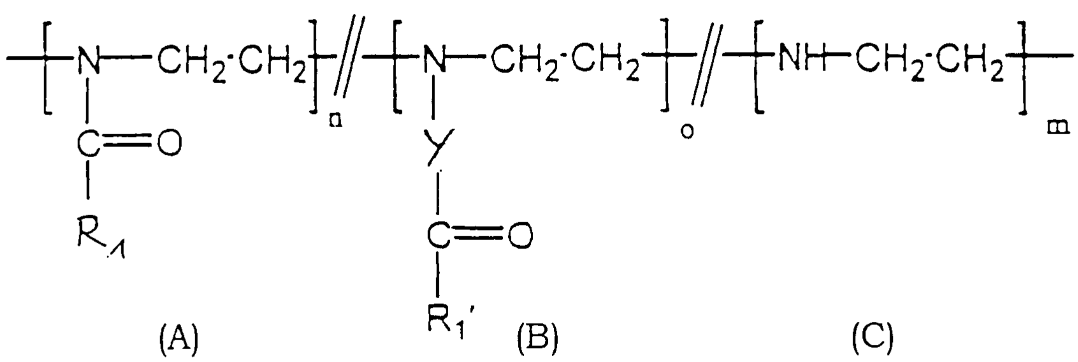

(A) (B) (C) (II),

worin bedeuten:

| | |
|---|---|
| $R^1$, $R_1$' und Y | dasselbe wie in den Ansprüchen 1 bis 3, |
| m | 2 bis 25, vorzugsweise 3 bis 10, |
| n | 5 bis 300, vorzugsweise 15 bis 100, |

und

o 2 bis 50, vorzugsweise 3 bis 20 und insbesondere 3 bis 10,

wobei die Doppelstriche // bedeuten, dass die Einheiten A, B und C in den Polymerketten statistisch verteilt oder in Form von Blöcken beliebiger Abfolge vorliegen.

7. Copolymere nach Anspruch 5 oder 6, in deren Formel $R_1$ Methyl oder Ethyl und $R_1'$ Vinyl, 1-Methylvinyl oder 1-Ethylvinyl bedeuten.

8. Copolymere nach einem oder mehreren der Ansprüche 1 bis 7, **dadurch gekennzeichnet, dass** es sich um statistische Copolymere handelt.

9. Copolymere nach einem oder mehreren der Ansprüche 1 bis 7, **dadurch gekennzeichnet, dass** es sich um Blockcopolymere handelt.

10. Copolymere nach einem oder mehreren der Ansprüche 5 und 7 bis 9 der Formel

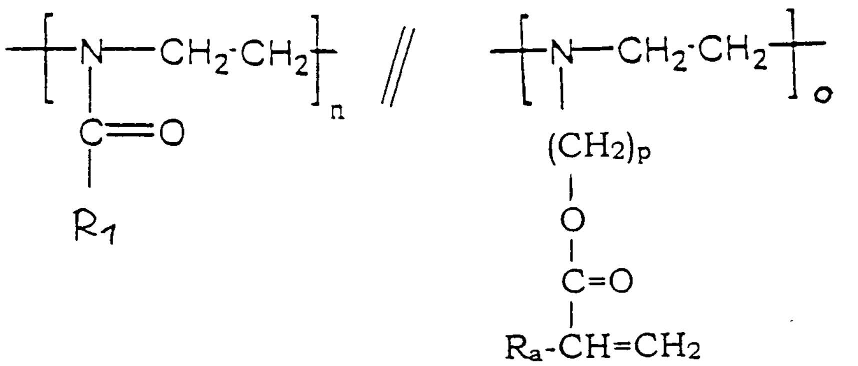

,

worin bedeuten:

$R_1$ dasselbe wie in den Ansprüchen 1 und 2,
$R_a$ H, Methyl oder Ethyl,
p eine ganze Zahl von 2 bis 7

und

n und o dasselbe wie in Anspruch 5,

wobei der Doppelstrich//bedeutet, dass die Einheiten A und B in den Polymerketten statistisch verteilt oder in Form von Blöcken beliebiger Abfolge vorliegen.

11. Copolymere nach einem oder mehreren der Ansprüche 6 bis 9 der Formel

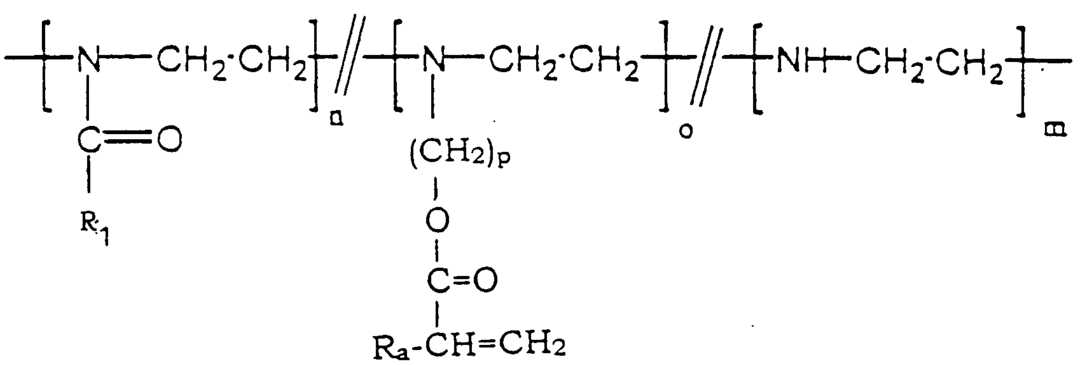

,

worin bedeuten:

$R_1$ dasselbe wie in den Ansprüchen 1 und 2,

$R_a$ H, Methyl oder Ethyl,

p eine ganze Zahl von 2 bis 7

und

m, n und o dasselbe wie in Anspruch 6,

wobei die Doppelstriche // bedeuten, dass die Einheiten A, B und C in den Polymerketten statistisch verteilt oder in Form von Blöcken beliebiger Abfolge vorliegen.

**12.** Homopolymere nach einem oder mehreren der Ansprüche 1 bis 3 mit ungesättigten Endgruppen, **gekennzeichnet durch** die Formel III,

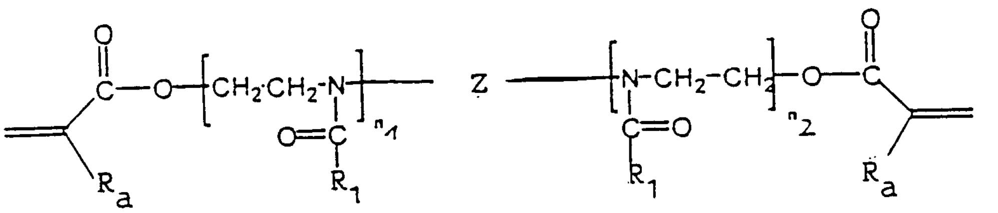

(III),

in der bedeuten:

$R_1$ dasselbe wie in den Ansprüchen 1 und 2,
$R_a$ H, Methyl oder Ethyl,
Z eine zweiwertige, von einem bifunktionellen kationischen Initiator abgeleitete Gruppe, insbesondere eine ungesättigte Gruppe und vorzugsweise die Gruppe $-CH_2-CH=CH-CH_2-$, und
$n_1$ und $n_2$ mittlere Polymerisationsgrade, deren Summe $n_1+n_2$ gleich n ist, wobei n 5 bis 300 bedeutet.

**13.** Homopolymere nach Anspruch 12 der Formel IV,

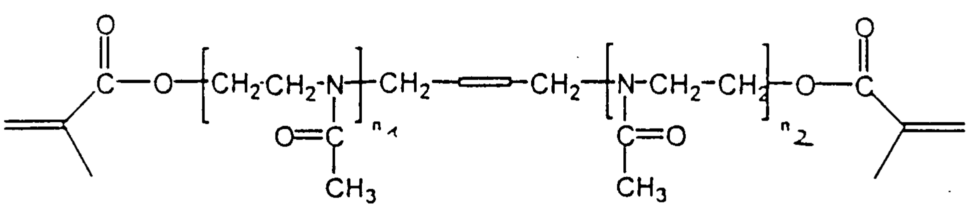

(IV)

mit $n_1$ und $n_2$ wie in Anspruch 12.

**14.** Copolymere nach einem oder mehreren der Ansprüche 1 bis 3 mit ungesättigten Endgruppen, **gekennzeichnet durch** die Formel V,

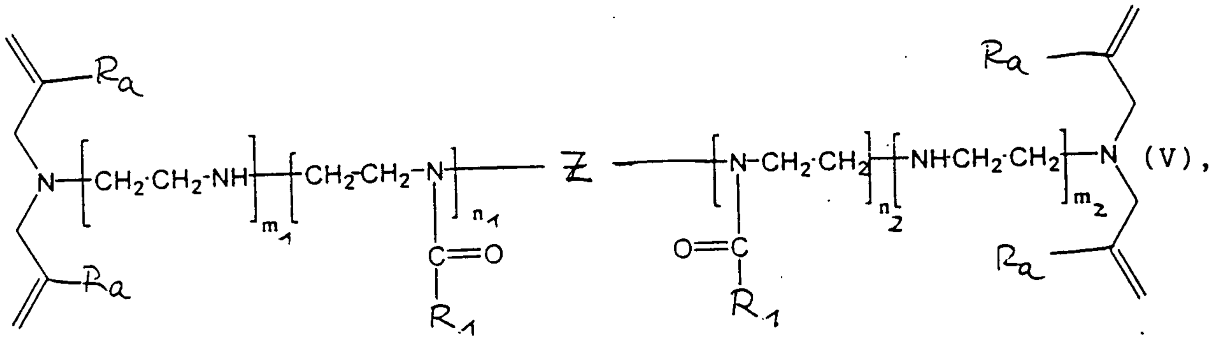

in der bedeuten:

| | |
|---|---|
| $R_1$ | dasselbe wie in den Ansprüchen 1 und 2, |
| $R_a$ | H, Methyl oder Ethyl, |
| Z | eine zweiwertige, von einem bifunktionellen kationischen Initiator abgeleitete Gruppe, insbesondere eine ungesättigte Gruppe und vorzugsweise die Gruppe $-CH_2-CH=CH-CH_2-$, |
| $m_1$ und $m_2$ | mittlere Polymerisationsgrade, deren Summe $m_1+m_2$ gleich m ist, wobei m 2 bis 25 bedeutet, und |
| $n_1$ und $n_2$ | mittlere Polymerisationsgrade, deren Summe $n_1+n_2$ gleich n ist, wobei n 5 bis 300 bedeutet. |

**15.** Copolymere nach Anspruch 14 der Formel VI

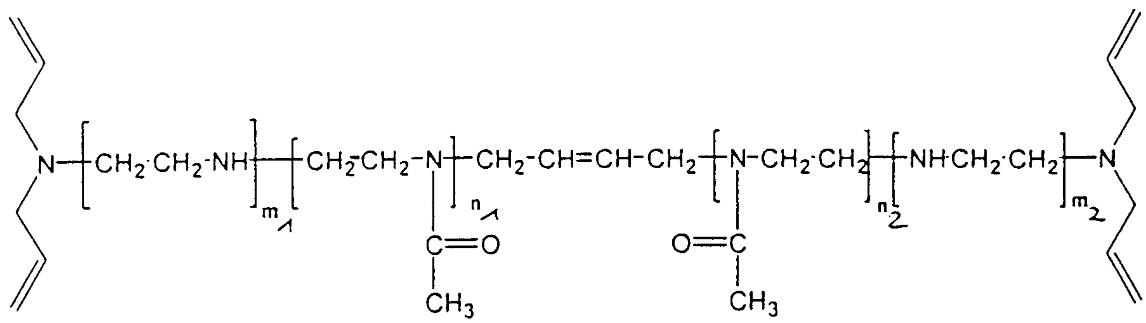

(VI)

mit $m_1$, $m_2$, $n_1$ und $n_2$ wie in Anspruch 14.

**16.** Copolymere nach einem oder mehreren der Ansprüche 1 bis 3 der Formel VII,

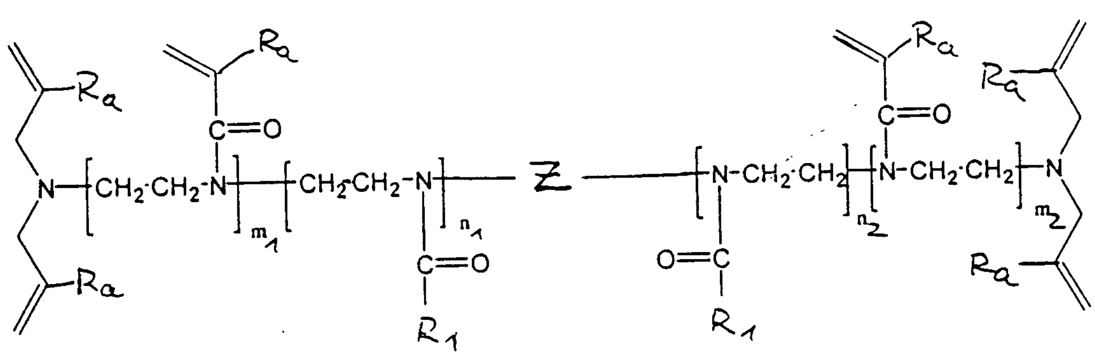

(VII),

in der bedeuten:

| | |
|---|---|
| $R_1$ | dasselbe wie in den Ansprüchen 1 und 2, |
| $R_a$ | H, Methyl oder Ethyl, |
| Z | eine zweiwertige, von einem bifunktionellen kationischen Initiator abgeleitete Gruppe, insbesondere eine ungesättigte Gruppe und vorzugsweise die Gruppe $-CH_2-CH=CH-CH_2-$, |
| $m_1$ und $m_2$ | mittlere Polymerisationsgrade, deren Summe $m_1+m_2$ gleich m ist, wobei m 2 bis 25 bedeutet, und |
| $n_1$ und $n_2$ | mittlere Polymerisationsgrade, deren Summe $n_1+n_2$ gleich n ist, wobei n 5 bis 300 bedeutet. |

**17.** Homopolymere und Copolymere nach den Ansprüchen 12, 14 und 16, **dadurch gekennzeichnet, dass** die zweiwertige Gruppe Z eine Gruppe der Formel $-CH_2-R-CH_2-$ ist, wobei R unter bifunktionellen Alkylgruppen, Arylgruppen, Alkengruppen, Alkingruppen, Alkylarylgruppen, Heteroarylgruppen, Alkylethergruppen und Alkylestergruppen ausgewählt ist.

**18.** Homopolymere und Copolymere nach Anspruch 17, **gekennzeichnet durch** eine Gruppe Z der Formel

$$-CH_2-CH=CH-CH_2- ,$$

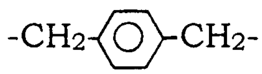
,

$$-CH_2-(CH_2)_{1-4}-CH_2-$$

oder

$$-CH_2-C\equiv C-CH_2-$$

**19.** Copolymere nach Anspruch 16 der Formel VIII

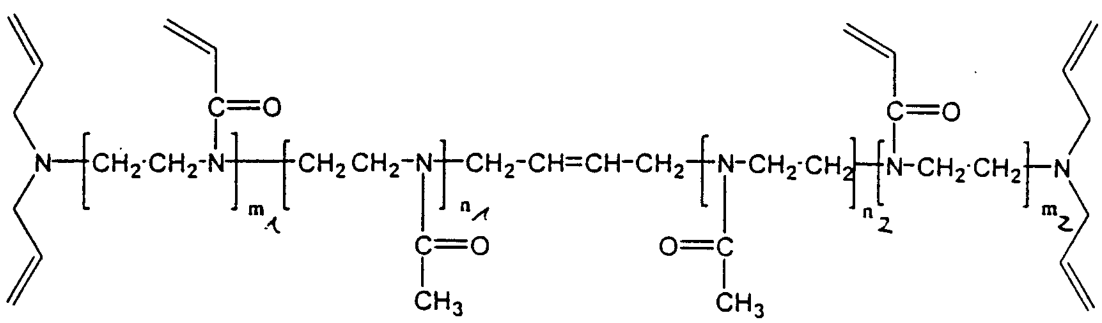
(VIII)

mit $m_1$, $m_2$, $n_1$ und $n_2$ wie in Anspruch 16.

**20.** Homopolymere und Copolymere nach einem oder mehreren der Ansprüche 1 bis 19, **dadurch gekennzeichnet, dass** sie vernetzt sind.

**21.** Homopolymere und Copolymere nach Anspruch 18, **dadurch gekennzeichnet, dass** sie über darin enthaltene ungesättigte Gruppen radikalisch vernetzt sind.

**22.** Vernetzte Homopolymere und Copolymere nach Anspruch 20 oder 21, **dadurch gekennzeichnet, dass** sie über Gruppen $R_1'$, Z und/oder ungesättigte Endgruppen vernetzt sind.

**23.** Verfahren zur Herstellung von Homopolymeren der Formel

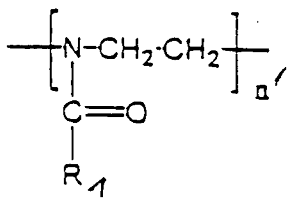

,

worin bedeuten:

$R_1$ dasselbe wie in den Ansprüchen 1 und 2 und

n' den mittleren Polymerisationsgrad, der vorzugsweise im Bereich von 5 bis 500 liegt,

**gekennzeichnet durch** polymeranaloge Umsetzung eines Polyethylenimins der Formel

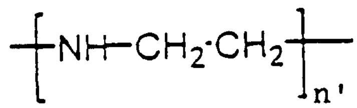

mit n' wie oben
mit einer Verbindung der Formel

$$R_1\text{-CO-X}$$

mit X = Halogen, insbesondere Cl, in Gegenwart eines Säureakzeptors.

**24.** Verfahren zur Herstellung von Homopolymeren nach den Ansprüchen 1 bis 3, 12 und 13, **gekennzeichnet durch** kationische ringöffnende Polymerisation eines 2-substituierten 2-Oxazolins M nach folgender Reaktionsgleichung:

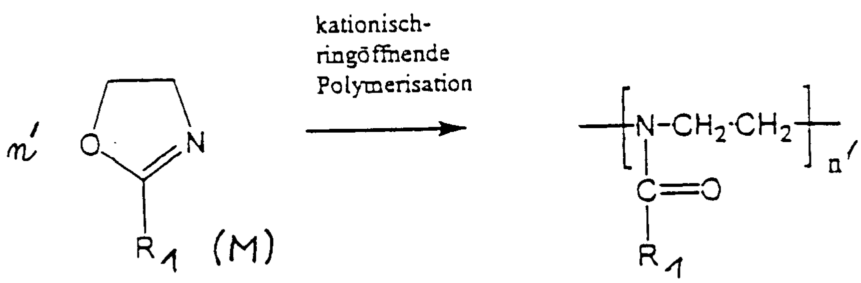


,

wobei bedeuten:

$R_1$ dasselbe wie in den Ansprüchen 1 und 2
und
n' den mittleren Polymerisationsgrad, der vorzugsweise im Bereich von 5 bis 500 liegt,

unter Verwendung eines kationischen Initiators.

**25.** Verfahren zur Herstellung von Homopolymeren nach den Ansprüchen 1 bis 3, **gekennzeichnet durch** kationische ringöffnende Polymerisation eines 2-substituierten 2-Oxazolins N nach folgender Reaktionsgleichung:

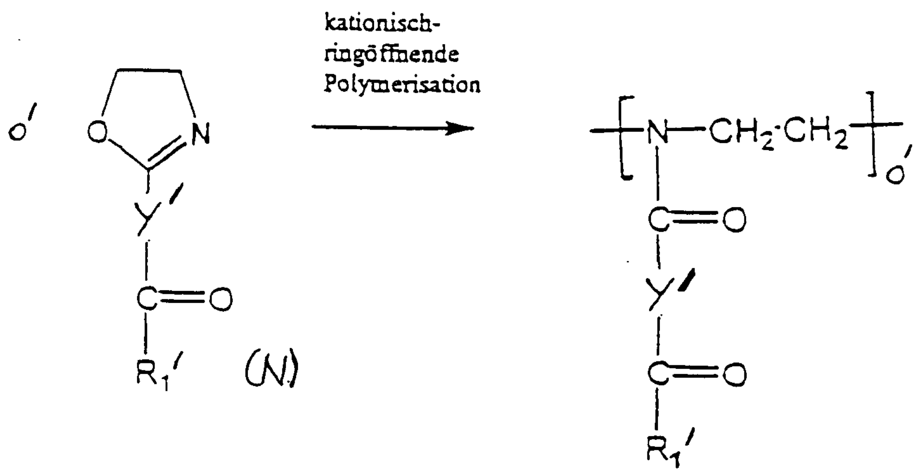

(N)
,

wobei bedeuten:

$R_1$' dasselbe wie in den Ansprüchen 1 und 3,
Y' eine Einfachbindung
oder
eine Gruppe der Formel $-(CH_2)_p$O- mit p = 2 bis 7, die mit ihrem C-Ende am 2-Oxazolinring bzw. an der am N-Atom gebundenen CO-Gruppe gebunden ist,
und
o' den mittleren Polymerisationsgrad, der vorzugsweise im Bereich von 5 bis 500 liegt,

unter Verwendung eines kationischen Initiators.

**26.** Verfahren zur Herstellung von Homopolymeren der Formel

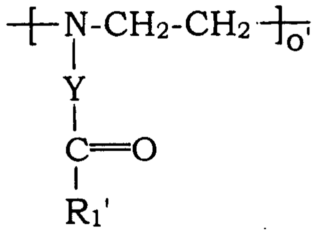
,

worin bedeuten:

$R_1$' und Y dasselbe wie in den Ansprüchen 1 bis 3 und
o' den mittleren Polymerisationsgrad, der vorzugsweise im Bereich von 5 bis 500 liegt,

**gekennzeichnet durch** polymeranaloge Umsetzung eines Polyethylenimins der Formel

$$-[N\text{-}CH_2\text{-}CH_2]_{o'}-$$

mit o' wie oben
mit einer Verbindung der Formel

$R_1$'-CO-Y-X

mit X = Halogen, insbesondere Cl,
in Gegenwart eines Säureakzeptors.

**27.** Verfahren zur Herstellung von Copolymeren der Formel I nach Anspruch 5,
**gekennzeichnet durch**
kationische ringöffnende Polymerisation
eines oder mehrerer 2-substituierter 2-Oxazoline M der Formel

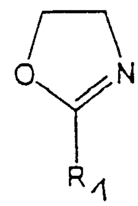 (M)

und eines oder mehrerer 2-substituierter 2-Oxazoline N der Formel

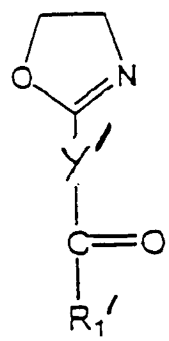 , (N)

worin $R_1$ und $R_1$' dasselbe wie in den Ansprüchen 1 bis 3 und Y' dasselbe wie in Anspruch 25 bedeuten,
in Gegenwart eines kationischen Initiators.

**28.** Verfahren zur Herstellung von Copolymeren der Formel II nach Anspruch 6, **gekennzeichnet durch** polymeranaloge Umsetzung eines Polyethylenimins der Formel

$$\left[ N\text{-}CH_2\text{-}CH_2 \right]_{m+n+o}$$

mit
einer oder mehreren Verbindungen der Formel

$$R_1\text{-CO-X}$$

und einer oder mehreren Verbindungen der Formel

$$R_1'\text{-CO-Y-X} ,$$

wobei bedeuten:

| | |
|---|---|
| $R_1$, $R_1$' und Y | dasselbe wie in den Ansprüchen 1 bis 3, |
| m, n und o | dasselbe wie in Anspruch 6 und |

X ein Halogen, insbesondere Cl,

in Gegenwart eines Säureakzeptors.

**29.** Verfahren zur Herstellung von Copolymeren der Formel II nach Anspruch 6, **gekennzeichnet durch**

(i) partielle Hydrolyse eines N-substituierten Poly-2-oxazolins der Formel

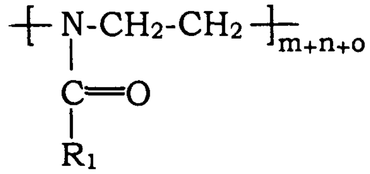

mit $R_1$ wie in den Ansprüchen 1 und 2 und m, n und o wie in Anspruch 6, mit einer Base, insbesondere wässeriger NaOH oder KOH, nach der Gleichung

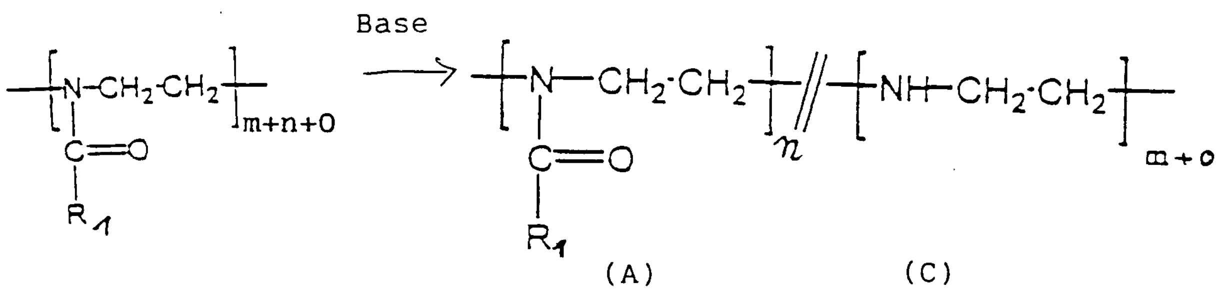


(A) (C)

und
(ii) Umsetzung gebildeter Polyethylenimin-Einheiten C mit einer oder mehreren Verbindungen der Formel

$$R_1'\text{-CO-Y-X}$$

mit X = Halogen, insbesondere Cl,
und Y wie in Anspruch 1 oder 3
in Gegenwart eines Säureakzeptors, insbesondere Pyridin,

wobei der Doppelstrich // bedeutet, dass die Einheiten A und die Ethylenimin-Einheiten C in den Polymerketten des in Schritt (i) erhaltenen partiell hydrolysierten Polymers statistisch verteilt oder in Form von Blöcken beliebiger Abfolge vorliegen.

**30.** Verfahren nach Anspruch 28 oder 29, **gekennzeichnet durch** Verwendung von Acryloylchlorid und/oder Methacryloylchlorid und/oder Ethacryloylchlorid als Verbindung der Formel $R_1'$-CO-X (Y = Einfachbindung) sowie eines N-substituierten Poly-2-oxazolins, das Endgruppen, insbesondere ungesättigte Endgruppen, aufweist, vorzugsweise Diallylamino- oder Acryloyloder Methacryloyl-Endgruppen.

**31.** Verfahren von Homopolymeren der Formel III bzw. IV nach Anspruch 12 oder 13, **gekennzeichnet durch**

- kationische ringöffnende Polymerisation eines 2-substituierten 2-Oxazolins M in Gegenwart eines bifunktionellen kationischen Initiators zu einem lebenden Polymer nach

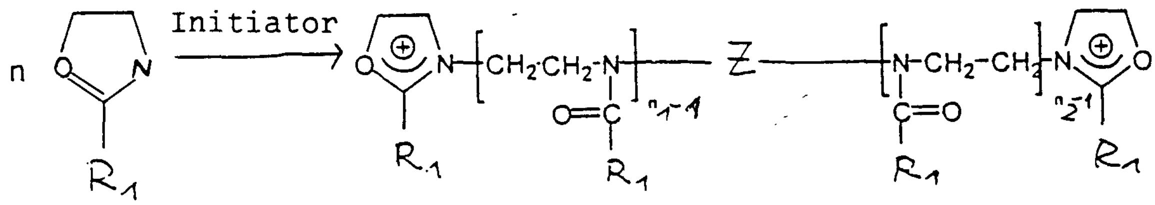


(M)

und

- Endgruppen-Funktionalisierung **durch** Umsetzung des lebenden Polymers mit einer nucleophilen Verbindung der Formel

$$H_2C{=}CH(R_a){-}COOH$$

oder eines aktiven Derivats dieser Verbindung zum Polymer mit ungesättigten Endgruppen der Formel III,

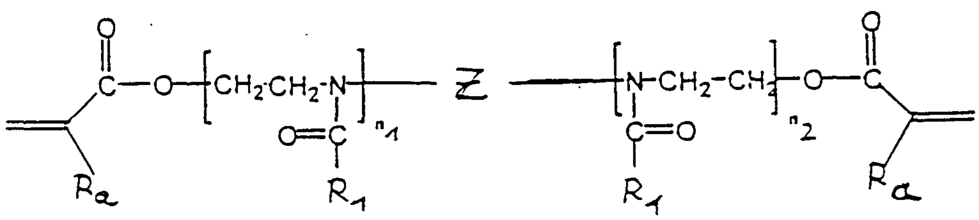
(III),

wobei $R_1$, $R_a$, Z und $n_1$ und $n_2$ dasselbe bedeuten wie in Anspruch 12.

**32.** Verfahren nach Anspruch 31, **gekennzeichnet durch**

- Verwendung von 2-Methyl-2-oxazolin als 2-substituiertes 2-Oxazolin M,
- Verwendung von trans-1,4-Dibrom-2-buten als Initiator und
- Verwendung von Methacrylsäure als nucleophile Verbindung in Gegenwart einer Base, insbesondere Triethylamin.

**33.** Verfahren nach einem oder mehreren der Ansprüche 23 bis 32, **dadurch gekennzeichnet, dass** das erhaltene Homopolymer oder Copolymer in Gegenwart eines radikalischen Initiators vernetzt wird.

**34.** Verfahren nach Anspruch 33, **dadurch gekennzeichnet, dass** die Vernetzung thermisch oder photochemisch, insbesondere durch UV-Bestrahlung, in einem Lösungsmittel durchgeführt wird.

**35.** Verfahren nach Anspruch 34, **dadurch gekennzeichnet, dass** als Lösungsmittel Wasser, Aceton oder Ethanol verwendet wird.

**36.** Verfahren nach Anspruch 33 oder 34, **dadurch gekennzeichnet, dass** das Polymer in Lösung auf ein Substrat, insbesondere ein Flächengebilde wie einVlies, aufgebracht oder durch Imprägnieren eingebracht und anschließend photochemisch vernetzt wird.

**37.** Verwendung der Homopolymeren und Copolymeren nach den Ansprüchen 1 bis 22 als Sorbentien oder zur Herstellung von Sorbentien und Artikeln zur Absorption von Wasser und wässerigen Flüssigkeiten, insbesondere Körperflüssigkeiten wie Blut und Sekrete, und Körperausscheidungen, wie Urin und Kot.

**38.** Verwendung der Homopolymeren und Copolymeren nach den Ansprüchen 1 bis 22 als Absorber zur Herstellung von oder in Klärmitteln für Flüssigkeiten, insbesondere in der Nahrungsund Genussmittelindustrie und der chemischen Industrie, Verbandmaterialien, Hygieneartikeln, insbesondere Babywindeln, Damenbinden, Slipeinlagen, Tampons, Inkontinenzprodukten, Pflanzensubstraten und Verpackungen und Lagerschalen für flüssigkeitsabsondernde Nahrungsmittel.

**39.** Wässerige Flüssigkeiten absorbierende Artikel, insbesondere Flächengebilde, die ein Substrat sowie ein oder mehrere Polymere nach den Ansprüchen 1 bis 22, die auf dem oder im Substrat vorliegen, aufweisen.

**40.** Absorbierende Artikel nach Anspruch 39, **dadurch gekennzeichnet, dass** das Polymer durch Vernetzung am bzw. im Substrat gebunden ist.

**41.** Absorbierende Artikel nach Anspruch 39 oder 40, **gekennzeichnet durch** Faserstoffe, Vliese, Textilgewebe oder Granulate als Substrat.

**42.** Absorbierender Artikel (1) nach einem oder mehreren der Ansprüche 39 bis 41, der aufweist:

- eine flüssigkeitsundurchlässige Grundschicht (3), die der Bekleidung des Trägers zugewandt ist,
- eine luft- und flüssigkeitsdurchlässige Deckschicht (2), die dem Körper des Trägers zugewandt ist, und
- eine zwischen der Grundschicht (3) und der Deckschicht (2) vorgesehene flüssigkeitsabsorbierende Saugschicht (5), die einen Vliesstoff niederer Dichte enthält,

**dadurch gekennzeichnet, dass** der Vliesstoff ein oder mehrere vernetzte Polymere nach den Ansprüchen 20 bis 22 enthält, die durch Imprägnierung mit unvernetztem oder vorvernetztem Polymer und anschließende Vernetzung am bzw. im Vliesstoff gebunden sind.

**43.** Absorbierender Artikel nach Anspruch 42, der ferner eine Verteilschicht (4) aufweist, die zwischen der Deckschicht (2) und der Saugschicht (5) angeordnet ist.

**44.** Absorbierender Artikel nach Anspruch 42 oder 43, **gekennzeichnet durch** einen hydrophilen Vliesstoff in der Saugschicht (5).

**45.** Absorbierender Artikel nach Anspruch 42 odeer 43, **gekennzeichnet durch** einen hydrophoben Vliesstoff in der Saugschicht (5).

**46.** Absorbierender Artikel nach einem oder mehreren der Ansprüche 42 bis 45, **dadurch gekennzeichnet, dass** die Saugschicht (5) mehrschichtig ist und aus einer ersten Kernschicht aus einem hydrophilen synthetischen Mikrofaservlies, die von einem Vliesstoff niederer Dichte umhüllt ist, und einer zweiten Kernschicht aus einem hydrophilen synthetischen Mikrofaservlies besteht, die mit einer weiteren Kernschicht aus hoch wasserspeicherndem Material in Kontakt ist, wobei eine oder mehrere Schichten der Saugschicht (5) ein oder mehrere Polymere nach den Ansprüchen 20 bis 22 enthalten.

**47.** Absorbierender Artikel nach Anspruch 46, **dadurch gekennzeichnet, dass** die weitere Kernschicht Cellulosefasern und/oder superabsorbierende Partikel und/oder superabsorbierende Fasern enthält.

**48.** Absorbierender Artikel nach einem oder mehreren der Ansprüche 44 bis 47, **dadurch gekennzeichnet, dass** der hydrophile Vliesstoff der Saugschicht (5) ein hydrophilierter Polypropylen-Meltblown-Vliesstoff ist.

**49.** Verfahren zur Herstellung der absorbierenden Artikel nach den Ansprüchen 39 bis 48, **gekennzeichnet durch** folgende Schritte:

(1) Aufbringen einer Lösung eines oder mehrerer unvernetzter oder vorvernetzter Polymerer nach einem der Ansprüche 1 bis 22 sowie eines radikalischen Initiators in einem Lösungsmittel auf ein Substrat, insbesondere eine für die Saugschicht (5) vorgesehene Schicht,
(2) Verdampfen des Lösungsmittels
und
(3) Vernetzung oder Nachvernetzung des Polymers auf bzw. im Substrat **durch** thermische und/oder photochemische Vernetzung, insbesondere **durch** UV-Bestrahlung, sowie gegebenenfalls
(4) Nachreaktion zur Vervollständigung der Vernetzung.

**50.** Verfahren nach Anspruch 49, **gekennzeichnet durch** Verwendung von Wasser, polaren Lösungsmitteln, insbesondere aliphatischen Alkoholen, wie Ethanol, oder Aceton als Lösungsmittel in Schritt (1).

**51.** Verfahren nach Anspruch 49 oder 50, **gekennzeichnet durch** Verwendung von Benzoylperoxid, AIBN, 2,2'-Azobis [2-(2-imidazolin-2-yl)-propan]-dihydrochlorid, Azobis(2-amidinopropan)-dihydrochlorid (ABAH) und/oder Azobis (cyanopentansäure) als Initiator.

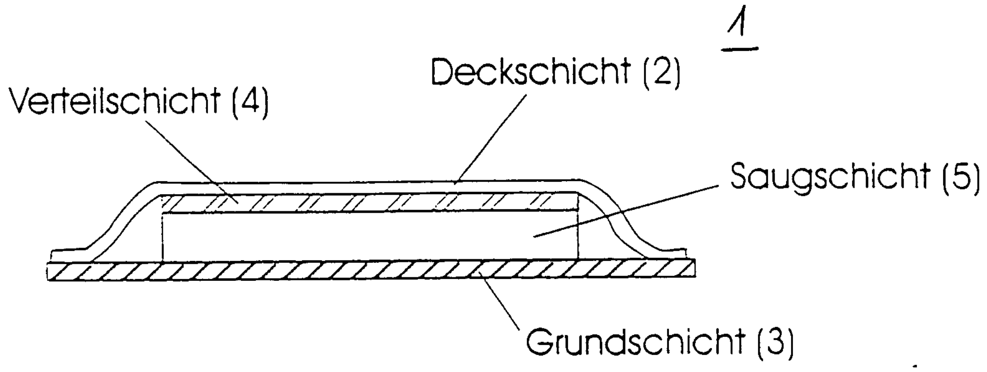
1
Deckschicht (2)
Verteilschicht (4)
Saugschicht (5)
Grundschicht (3)

Fig. 1

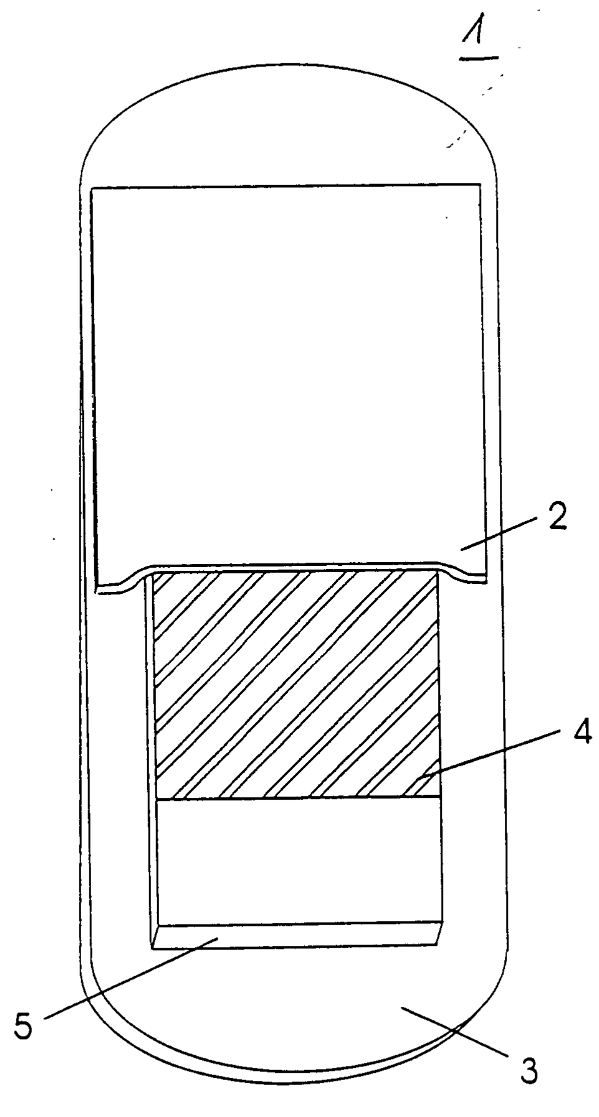
1
2
4
5
3

Fig. 2

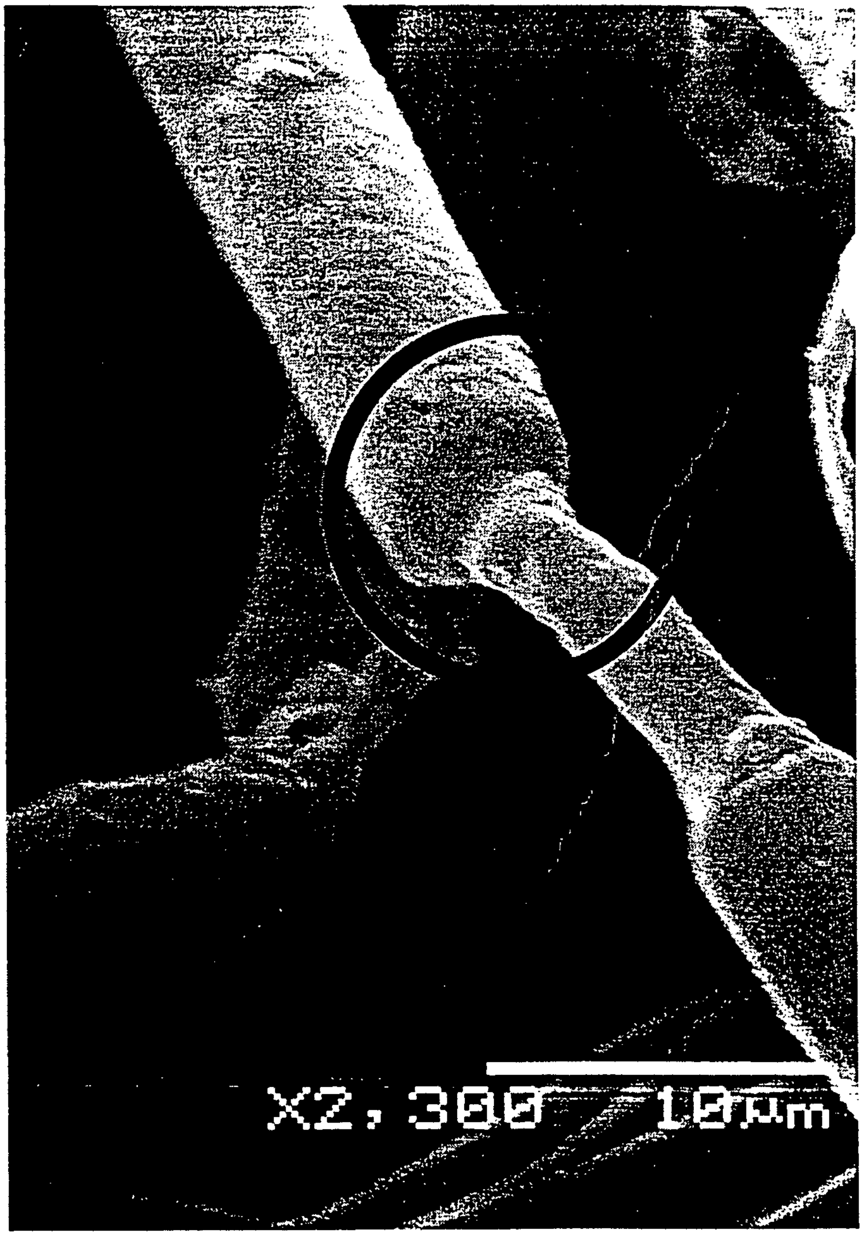


Fig. 3

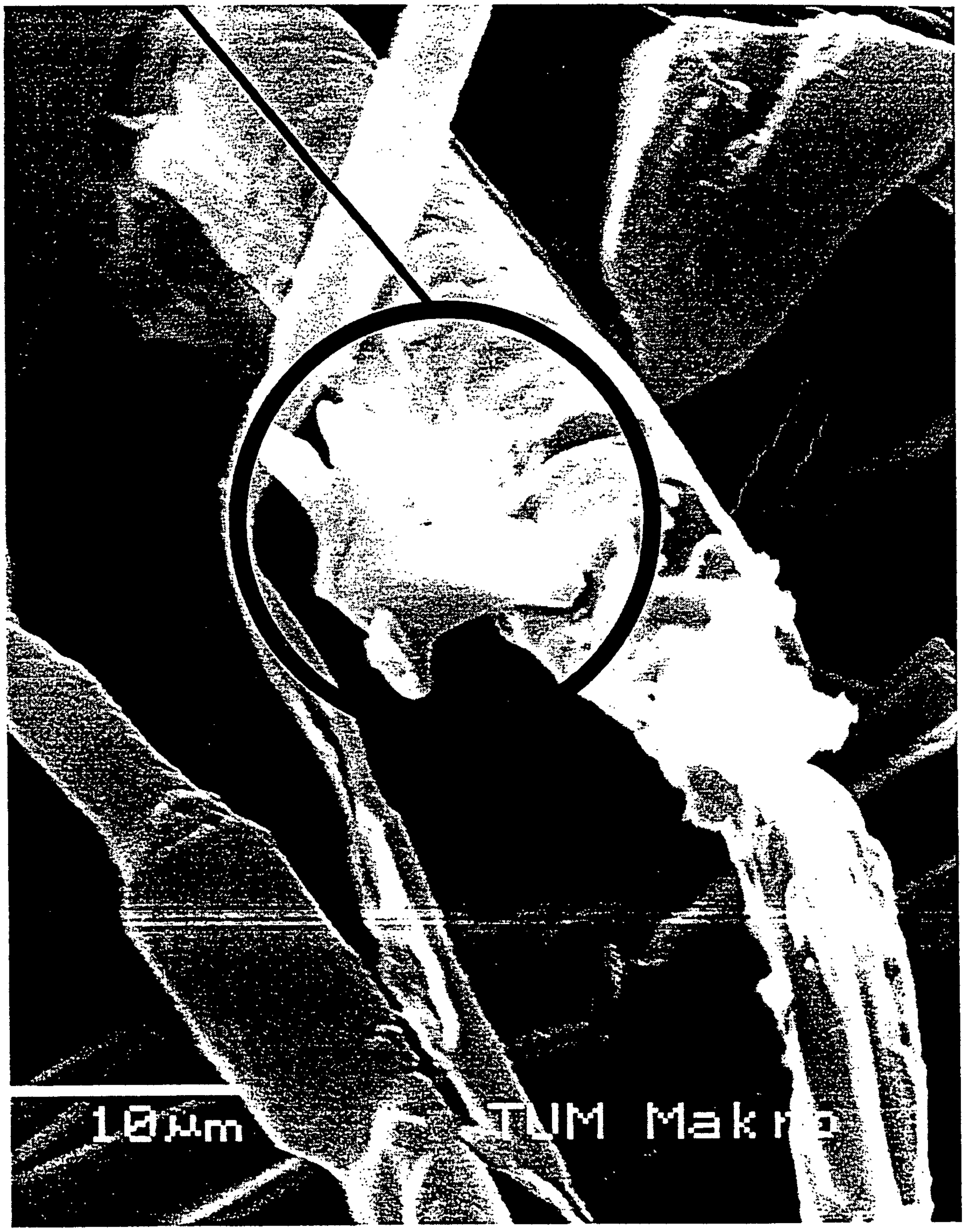


Fig. 4

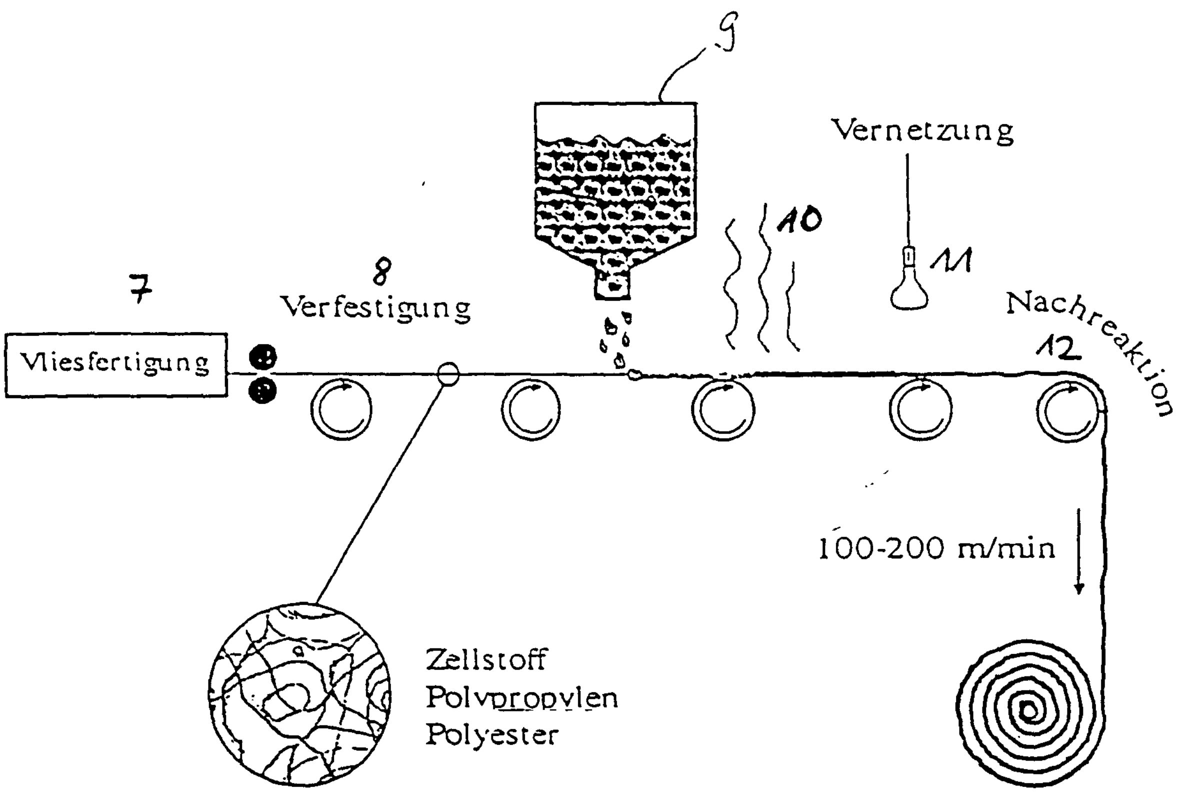
9
Vernetzung
10
11
7
8
Verfestigung
Nachreaktion
12
Vliesfertigung
100-200 m/min
Zellstoff
Polypropylen
Polyester

Fig. 5

Europäisches Patentamt

# EUROPÄISCHER RECHERCHENBERICHT

Nummer der Anmeldung
EP 02 02 5781

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | US 5 635 571 A (FRECHET ET AL.)<br>3. Juni 1997 (1997-06-03)<br>* Zusammenfassung; Ansprüche 1-10 *<br>--- | 1-11,<br>37-40 | C08G73/02<br>A61L15/26 |
| X | US 4 540 747 A (SAEGUSA ET AL.)<br>10. September 1985 (1985-09-10)<br>* Ansprüche 1-9 *<br>--- | 1-14 | |
| X | CH 497 487 A (ALLIED CHEMICAL CORPORATION)<br>15. Oktober 1970 (1970-10-15)<br>* Anspruch 1 *<br>--- | 1,32 | |
| A | DE 39 43 111 A (HENKEL KGAA)<br>4. Juli 1991 (1991-07-04)<br>* Ansprüche 1-14 *<br>----- | 1,12-22 | |

RECHERCHIERTE SACHGEBIETE (Int.Cl.7)
C08G
A61L

Der vorliegende Recherchenbericht wurde für alle Patentansprüche erstellt

| Recherchenort | Abschlußdatum der Recherche | Prüfer |
|---|---|---|
| DEN HAAG | 3. April 2003 | Glanddier, A |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 02 02 5781

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

03-04-2003

| Im Recherchenbericht angeführtes Patentdokument | Datum der Veröffentlichung | Mitglied(er) der Patentfamilie | Datum der Veröffentlichung |
|---|---|---|---|
| US 5635571 A | 03-06-1997 | US 5830948 A | 03-11-1998 |
| US 4540747 A | 10-09-1985 | JP 1801835 C | 12-11-1993 |
| | | JP 5007415 B | 28-01-1993 |
| | | JP 59230027 A | 24-12-1984 |
| | | JP 2018274 C | 19-02-1996 |
| | | JP 3067093 B | 21-10-1991 |
| | | JP 59230028 A | 24-12-1984 |
| | | DE 3421525 A1 | 13-12-1984 |
| CH 497487 A | 15-10-1970 | BE 666828 A | 03-11-1965 |
| | | CH 498897 A | 15-11-1970 |
| | | DE 1570213 A1 | 02-01-1970 |
| | | FR 1450751 A | 24-06-1966 |
| | | GB 1123678 A | 14-08-1968 |
| | | GB 1123674 A | 14-08-1968 |
| | | NL 6509052 A | 14-01-1966 |
| | | US 3483141 A | 09-12-1969 |
| DE 3943111 A | 04-07-1991 | DE 3943111 A1 | 04-07-1991 |
| | | WO 9109897 A2 | 11-07-1991 |
| | | EP 0507790 A1 | 14-10-1992 |
| | | JP 5502685 T | 13-05-1993 |

EPO FORM P0461

Für nähere Einzelheiten zu diesem Anhang : siehe Amtsblatt des Europäischen Patentamts, Nr.12/82